# NEW EUROPEAN PATENT SPECIFICATION

(11) **EP 0 640 578 B2**
(45) Date of publication and mention of the opposition decision: **27.06.2001**
(45) Mention of the grant of the patent: 05.11.1997
(21) Application number: 94306361.0
(22) Date of filing: 30.08.1994
(51) Int. Cl.: C07C 22/08, C07C 23/18, C07C 25/00, C09K 19/08

(54) **Novel liquid crystalline compound having a 1,1,2,3,3,3,-hexafluoropropyl group and liquid crystal composition**
Neue 1,1,2,3,3,3-Hexafluoropropylgruppe enthaltende Flüssigkristallverbindungen und Flüssigkristallzusammensetzungen
Nouveaux composés cristal liquide contenant un groupe 1,1,2,3,3,3-hexafluoropropyle et compositions les contenant

(30) Priority: 30.08.1993 JP 21458093; 18.04.1994 JP 7895594
(43) Date of publication of application: 01.03.1995
(73) Proprietor: Chisso Corporation, Osaka-shi Osaka 530-0055 (JP)
(72) Inventor: Miyazawa, Kazutoshi, Ichihara-shi, Chiba-ken (JP); Goto, Yasuyuki, Ichihara-shi, Chiba-ken (JP); Matsui, Shuichi, Ichihara-shi, Chiba-ken (JP); Fujita, Atsuko, Midori-ku, Chiba-shi, Chiba-ken (JP); Tomi, Yoshitaka, Ichihara-shi, Chiba-ken (JP)
(74) Representative: Stein-Dräger, Christiane

(56) References cited:
- EP-A- 0 446 911
- WO-A-91/03450
- WO-A-93/03113
- DE-A- 4 137 401
- US-A- 5 122 297
- BERICHTE DER BUNSEN-GESELLSCHAFT, PHYSIKALISCHE CHEMIE vol. 97, no. 10 , October 1993 , WEINHEIM pages 1349 - 1355 E. BARTMANN 'Flüssigkristalle mit fluorhaltigen Alkylgruppen'

## Description

### 1. Field of the Invention

This invention relates to liquid crystalline compounds and liquid crystal compositions. More particularly, it relates to novel liquid crystalline compounds having a hexafluoropropyl group and liquid crystal compositions containing the same.

### 2. Description of the Related Art

Liquid crystal compositions, particularly nematic liquid crystal compositions, have been broadly used for various display materials such as displays for watches, electronic calculators, word processors, computer terminals or television, etc. Liquid crystal display elements using nematic liquid crystal compositions have three kinds of main driving modes of twisted nematic (hereinafter abbreviated to TN) mode, super-twisted nematic (hereinafter abbreviated to STN) mode and thin-film transistor drive mode (hereinafter abbreviated to TFT). Among these, TFT mode has a superior display capability; hence it has been used for televisions and large scale color display and broadening of its use applications has been most expected.

Liquid crystal compounds for TFT require the following characteristics of:
1) exhibiting nematic liquid crystal phase within a broad temperature range;
2) having a large dielectric anisotropy value (herein-after abbreviated to △ε);
3) having a large optical anisotropy value (hereinafter abbreviated to △n);
4) having a low viscosity; and
5) having a high voltage holding ratio (abbreviated hereinafter to VHR).

Various compounds have been proposed for satisfying these characteristics. As compounds having a high VHR, compounds containing fluorine atoms are superior, and research for liquid crystal compounds containing fluorine atoms has been extensively carried out. For example, compounds having 3,4-difluorobenzene core at the terminal of the molecule (DE 3042391), and compounds having 3,4,5-trifluorobenzene core aiming at a larger Δε (USP 5032313) have been known, but they are unsuitable due to their very low clearing points. Further, compounds having introduced trifluoromethyl group, trifluoromethoxy group or difluoromethoxy group at the terminal of the molecules (DE 4027840) have been known, but they have drawbacks that the clearing point is still low and the An is still small. Further, in recent years, a compound having 1,1,2,2-tetrafluoroethoxy group at its terminal (DE 4142519) and a compound having 2,2,2-tri-fluoroethoxy group at its terminal (WO 93/3113) have been reported, but they have drawbacks that their clearing points are low and moreover have no sufficiently large Δε; hence they are insufficient in the aspects of characteristics.

In order to obtain a compound exhibiting a larger Δε, an example of a compound having introducing a cyano group having a large dipole moment has been reported (Japanese patent application laid-open Nos. Sho 62-103057 and Sho 63-216858), but the viscosity not only increases, but also the VHR lowers; thus it was difficult to use them as liquid crystal compounds for TFT.

Berichte der Bunsengesellschaft, Physikalische Chemie, vol. 97, no 10, October 1993, pages 1349-1355 describes liquid crystals with fluorine-containing alkyl groups, and indicates that the paper was presented at the 92nd Annual Meeting of the Deutsche Bunsen-Gesellschaft Für Physikalische Chemie in May 1993.

### SUMMARY OF THE INVENTION

In order to solve the above problems, the present inventors have made extensive research, for providing liquid crystal compounds exhibiting a nematic liquid crystal phase within a broad temperature range and having a large △ε and △n, a low viscosity and a high VHR, and as a result, have completed the present invention.

The present invention resides in the following aspects.
(1) A liquid crystalline compound expressed by the formula (1) 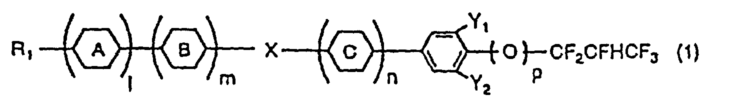 wherein R₁ represents an alkyl group of 1 to 12 carbon atoms and one CH₂ group or two CH₂ groups not adjacent to each other in the alkyl group may be replaced by group(s) selected from among oxygen atom, -CO-group, -O-CO- group, -CO-O- group and -CH=CH- group; X represents a covalent bond, -CH₂CH₂-, -CH=CH-, -C=C-, -CH₂O- , -OCH₂-, -CO-O- or -O-CO-; Y₁ and Y₂ each independently represent hydrogen atom, fluorine atom, chlorine atom or methyl group; rings A, B and C each independently represent a benzene ring or a cyclohexane ring and these rings may be substituted by a halogen atom(s) or a methyl group(s): and ℓ, m, n and p each independently represent 1 or 0, but ℓ+m is one or more,*
(2) A liquid crystalline compound according to item (1), wherein R₁ represents an alkyl group of 1 to 12 carbon atoms and one CH₂ group or two CH₂ groups thereof not adjacent to each other in the alkyl group may be replaced by oxygen atom or -CH₂=CH- group; X represents a covalent bond, -CH₂CH₂- or -C=C-; and Y₁ and Y₂ each independently represent hydrogen atom or fluorine atom.
(3) A liquid crystalline compound according to item (2), wherein the R₁ represents an alkyl group of 1 to 12 carbon atoms.
(4) A liquid crystalline compound according to item (3), wherein the ring C represents a benzene ring or a benzene ring substituted by halogen atom or methyl group.
(5) A liquid crystalline compound according to item (3), wherein the ring C represents a cyclohexane ring or a cyclohexane ring substituted by halogen atom or methyl group.
(6) A liquid crystalline compound according to item (2), wherein the R₁ represents an alkyl group of 1 to 12 carbon atoms and one CH₂ group or two CH₂ groups not adjacent to each other in the alkyl group is replaced by oxygen atom.
(7) A liquid crystalline compound according to item (6), wherein the ring C represents benzene ring or a benzene ring substituted by halogen atom or methyl group.
(8) A liquid crystalline compound according to item (6), wherein the ring C represents cyclohexane ring or a cyclohexane ring substituted by halogen atom or methyl group.
(9) A liquid crystal composition comprising at least two components at least one of which is a liquid crystalline compound set forth in any one of items (1) to (8).

* with the proviso that when p=1, then neither Y₁ nor Y₂ represent a fluorine atom.

### DETAILED DESCRIPTION OF PREFERRED EMBODIMENTS

The liquid crystalline compound expressed by the formula (1) of the present invention is characterized by having a group expressed by the following formula at the terminal of the molecule; 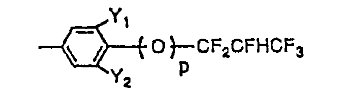 wherein Y₁, Y₂ and p are as defined above. Hereinafter the above formula will be referred to as Q.

This Q more concretely includes the following six groups:
3,5-disubstituted-4-(1,1,2,3,3,3-hexafluoropropoxy)phenyl group,
3-substituted-4-(1,1,2,3,3,3-hexafluoropropoxy)phenyl group,
4-(1,1,2,3,3,3-hexafluoro-propoxy)phenyl group,
3,5-disubstituted-4-(1,1,2,3,3,3-hexafluoropropyl)phenyl group,
3-substituted-4-(1,1,2,3,3,3-hexafluoropropyl)phenyl group, and
4-(1,1,2,3,3,3-hexafluoropropyl)phenyl group.

The liquid crystalline compound expressed by the formula (1) of the present invention characteristically having the Q is roughly classified in accordance with the kind of X in the formula, as follows:

In the case where X in the formula represents a covalent bond,

R₁-Cy-Q (1aa)

R₁-Ph-Q (1AB)

R₁-Cy-Cy-Q (1AC)

R₁-Cy-Ph-Q (1AD)

R₁-Ph-Cy-Q (1AE)

R₁-Ph-Ph-Q (1AF)

R₁-Cy-Cy-Cy-Q (1ag)

R₁-Cy-Cy-Ph-Q (1ah)

R₁-Cy-Ph-Cy-Q (1ai)

R₁-Ph-Cy-Cy-Q (1aj)

R₁-Cy-Ph-Ph-Q (1ak)

R₁-Ph-Cy-Ph-Q (1al)

R₁-Ph-Ph-Cy-Q (1am)

R₁-Ph-Ph-Ph-Q (1AN)

In the case of X = -CH₂CH₂-.

R₁-Cy-CH₂CH₂-Q (1ba)

R₁-Ph-CH₂CH₂-Q (1bb)

R₁-Cy-CH₂CH₂Cy-Q (1bc)

R₁-Cy-CH₂CH₂-Ph-Q (1bd)

R₁-Ph-CH₂CH₂-Cy-Q (1be)

R₁-Ph-CH₂CH₂-Ph-Q (1bf)

R₁-Cy-Cy-CH₂CH₂-Q (1bg)

R₁-Cy-Ph-CH₂CH₂-Q (1bh)

R₁-Ph-Cy-CH₂CH₂-Q (1bi)

R₁-Ph-Ph-CH₂CH₂-Q (1bj)

R₁-Cy-Cy-CH₂CH₂-Cy-Q (1bk)

R₁-Cy-Cy-CH₂CH₂-Ph-Q (1bl)

R₁-Cy-Ph-CH₂CH₂-Cy-Q (1bm)

R₁-Ph-Cy-CH₂CH₂-Cy-Q (1bn)

R₁-Cy-Ph-CH₂CH₂-Ph-Q (1bo)

R₁-Ph-Cy-CH₂CH₂-Ph-Q (1bp)

R₁-Ph-Ph-CH₂CH₂-Cy-Q (1bq)

R₁-Ph-Ph-CH₂CH₂-Ph-Q (1BR)

In the case of X = -CH=CH-,

R₁-Cy-CH=CH-Q (1ca)

R₁-Ph-CH=CH-Q (1cb)

R₁-Cy-CH=CH-Cy-Q (1cc)

R₁-Cy-CH=CH-Ph-Q (1cd)

R₁-Ph-CH=CH-Cy-Q (1ce)

R₁-Ph-CH=CH-Ph-Q (1cf)

R₁-Cy-Cy-CH=CH-Q (1CG)

R₁-Cy-Ph-CH=CH-Q (1ch)

R₁-Ph-Cy-CH=CH-Q (1ci)

R₁-Ph-Ph-CH=CH-Q (1cj)

R₁-Cy-Cy-CH=CH-Cy-Q (1ck)

R₁-Cy-Cy-CH=CH-Ph-Q (1cl)

R₁-Cy-Ph-CH=CH-Cy-Q (1cm)

R₁-Ph-Cy-CH=CH-Cy-Q (1cn)

R₁-Cy-Ph-CH=CH-Ph-Q (1co)

R₁-Ph-Cy-CH=CH-Ph-Q (1cp)

R₁-Ph-Ph-CH=CH-Cy-Q (1cq)

R₁-Ph-Ph-CH=CH-Ph-Q (1cr)

In the case of X = -C≡C-,

R₁-Cy-C≡C-Q (1da)

R₁-Ph-C≡C-Q (1db)

R₁-Cy-C≡C-Cy-Q (1dc)

R₁-Cy-C≡C-Ph-Q (1dd)

R₁-Ph-C≡C-Cy-Q (1de)

R₁-Ph-C≡C-Ph-Q (1df)

R₁-Cy-Cy-C≡C-Q (1dg)

R₁-Cy-Ph-C≡C-Q (1dh)

R₁-Ph-Cy-C≡C-Q (1di)

R₁-Ph-Ph-C≡C-Q (1dj)

R₁-Cy-Cy-C≡C-Cy-Q (1dk)

R₁-Cy-Cy-C≡C-Ph-Q (1dl)

R₁-Cy-Ph-C≡C-Cy-Q (1dm)

R₁-Ph-Cy-C≡C-Cy-Q (1dn)

R₁-Cy-Ph-C≡C-Ph-Q (1do)

R₁-Ph-Cy-C≡C-Ph-Q (1dp)

R₁-Ph-Ph-C≡C-Cy-Q (1dq)

R₁-Ph-Ph-C≡C-Ph-Q (1dr)

In the case of X = -CH₂O-,

R₁-Cy-CH₂O-Q (1ea)

R₁-Ph-CH₂O-Q (1eb)

R₁-Cy-CH₂O-Cy-Q (1ec)

R₁-Cy-CH₂O-Ph-Q (1ed)

R₁-Ph-CH₂O-Cy-Q (1ee)

R₁-Ph-CH₂O-Ph-Q (1ef)

R₁-Cy-Cy-CH₂O-Q (1eg)

R₁-Cy-Ph-CH₂O-Q (1eh)

R₁-Ph-Cy-CH₂O-Q (1ei)

R₁-Ph-Ph-CH₂O-Q (1ej)

R₁-Cy-Cy-CH₂O-Cy-Q (1ek)

R₁-Cy-Cy-CH₂O-Ph-Q (1el)

R₁-Cy-Ph-CH₂O-Cy-Q (1em)

R₁-Ph-Cy-CH₂O-Cy-Q (1en)

R₁-Cy-Ph-CH₂O-Ph-Q (1eo)

R₁-Ph-Cy-CH₂O-Ph-Q (1ep)

R₁-Ph-Ph-CH₂O-Cy-Q (1eq)

R₁-Ph-Ph-CH₂O-Ph-Q (1er)

In the case of X = -OCH₂-,

R₁-Cy-OCH₂-Q (1fa)

R₁-Ph-OCH₂-Q (1fb)

R₁-Cy-OCH₂-Cy-Q (1fc)

R₁-Cy-OCH₂-Ph-Q (1fd)

R₁-Ph-OCH₂-Cy-Q (1fe)

R₁-Ph-OCH₂-Ph-Q (1ff)

R₁-Cy-Cy-OCH₂-Q (1fg)

R₁-Cy-Ph-OCH₂-Q (1fh)

R₁-Ph-Cy-OCH₂-Q (1fi)

R₁-Ph-Ph-OCH₂-Q (1fj)

R₁-Cy-Cy-OCH₂-Cy-Q (1fk)

R₁-Cy-Cy-OCH₂-Ph-Q (1fl)

R₁-Cy-Ph-OCH₂-Cy-Q (1fm)

R₁-Ph-Cy-OCH₂-Cy-Q (1fn)

R₁-Cy-Ph-OCH₂-Ph-Q (1fo)

R₁-Ph-Cy-OCH₂-Ph-Q (1fp)

R₁-Ph-Ph-OCH₂-Cy-Q (1fq)

R₁-Ph-Ph-OCH₂-Ph-Q (1fr)

In the case of X = -CO-O-,

R₁-Cy-CO-O-Q (1ga)

R₁-Ph-CO-O-Q (1gb)

R₁-Cy-CO-O-Cy-Q (1gc)

R₁-Cy-CO-O-Ph-Q (1gd)

R₁-Ph-CO-O-Cy-Q (1ge)

R₁-Ph-CO-O-Ph-Q (1gf)

R₁-Cy-Cy-CO-O-Q (1gg)

R₁-Cy-Ph-CO-O-Q (1gh)

R₁-Ph-Cy-CO-O-Q (1gi)

R₁-Ph-Ph-CO-O-Q (1gj)

R₁-Cy-Cy-CO-O-Cy-Q (1gk)

R₁-Cy-Cy-CO-O-Ph-Q (1gl)

R₁-Cy-Ph-CO-O-Cy-Q (1gm)

R₁-Ph-Cy-CO-O-Cy-Q (1gn)

R₁-Cy-Ph-CO-O-Ph-Q (1go)

R₁-Ph-Cy-CO-O-Ph-Q (1gp)

R₁-Ph-Ph-CO-O-Cy-Q (1gq)

R₁-Ph-Ph-CO-O-Ph-Q (1gr)

In the case of X = -O-CO-,

R₁-Cy-O-CO-Q (1ha)

R₁-Ph-O-CO-Q (1hb)

R₁-Cy-O-CO-Cy-Q (1hc)

R₁-Cy-O-CO-Ph-Q (1hd)

R₁-Ph-O-CO-Cy-Q (1he)

R₁-Ph-O-CO-Ph-Q (1hf)

R₁-Cy-Cy-O-CO-Q (1hg)

R₁-Cy-Ph-O-CO-Q (1hh)

R₁-Ph-Cy-O-CO-Q (1hi)

R₁-Ph-Ph-O-CO-Q (1hj)

R₁-Cy-Cy-O-CO-Cy-Q (1hk)

R₁-Cy-Cy-O-CO-Ph-Q (1hl)

R₁-Cy-Ph-O-CO-Cy-Q (1hm)

R₁-Ph-Cy-O-CO-Cy-Q (1hn)

R₁-Cy-Ph-O-CO-Ph-Q (1ho)

R₁-Ph-Cy-O-CO-Ph-Q (1hp)

R₁-Ph-Ph-O-CO-Cy-Q (1hq)

R₁-Ph-Ph-O-CO-Ph-Q (1hr)

In the above formulas, R₁ and Q are as defined above; Cy represents a cyclohexane ring substituted by halogen atom or methyl group or not substituted; and Ph represents a benzene ring substituted by halogen atom or methyl group or not substituted.

Among these, concrete examples of liquid crystal compounds having particularly superior characteristics, namely those exhibiting a nematic liquid crystal phase within a broad temperature range, and having a large △ε and △n, a low viscosity and a high VHR, are the following (1aa-a) to (1hh-a):

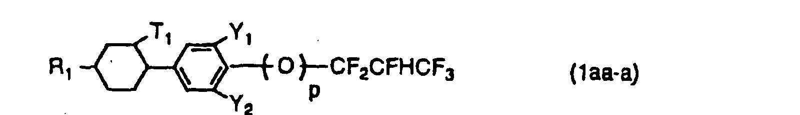

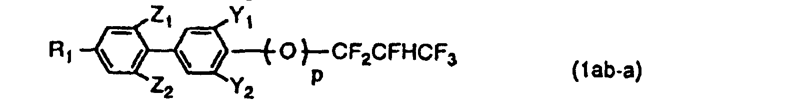

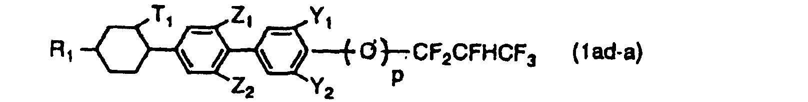

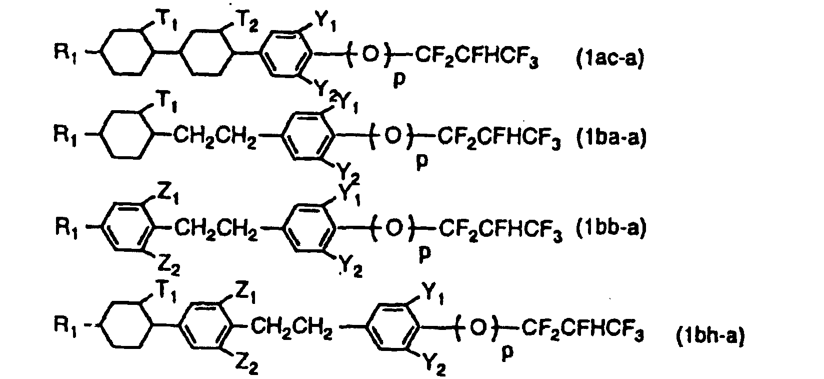

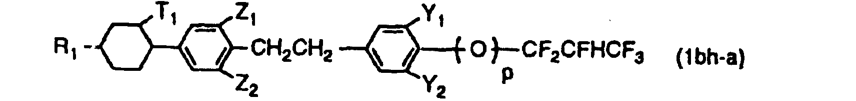

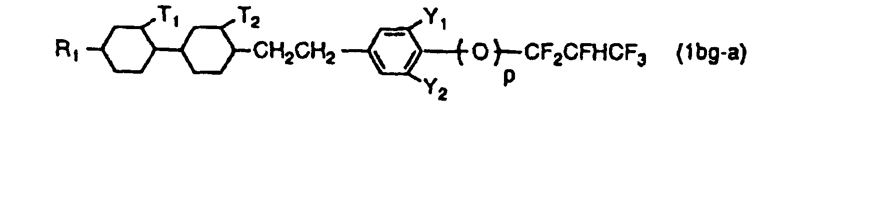

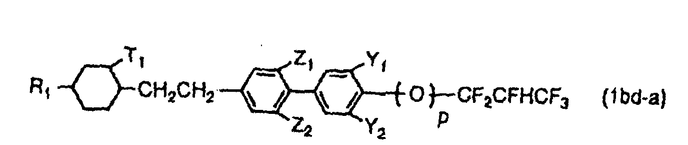

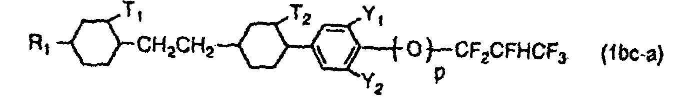

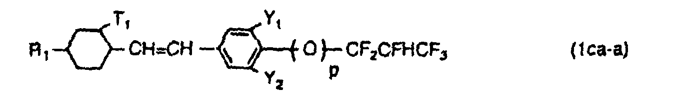

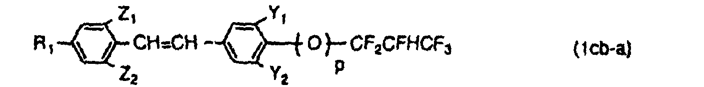

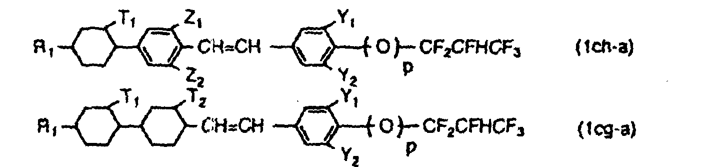

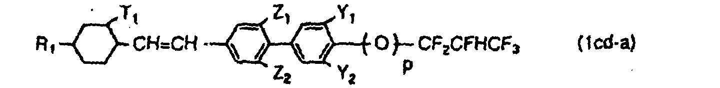

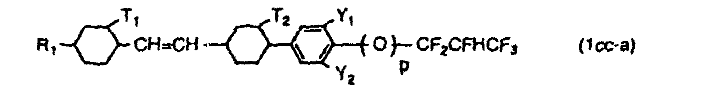

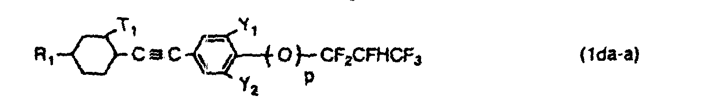

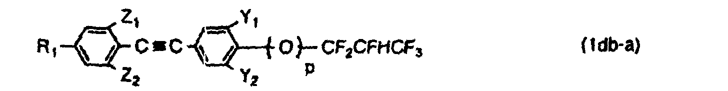

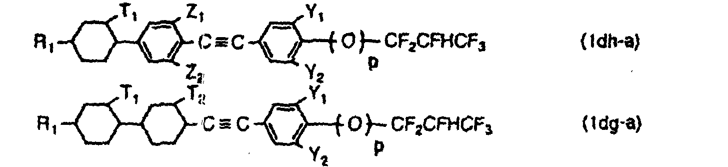

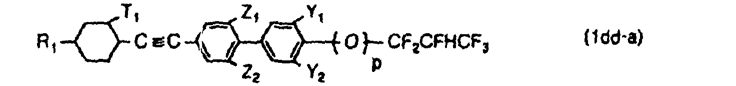

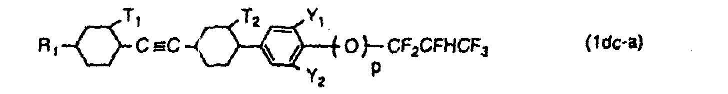

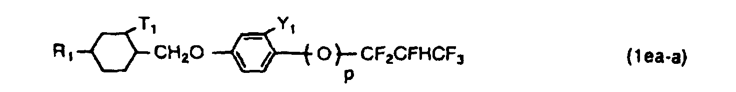

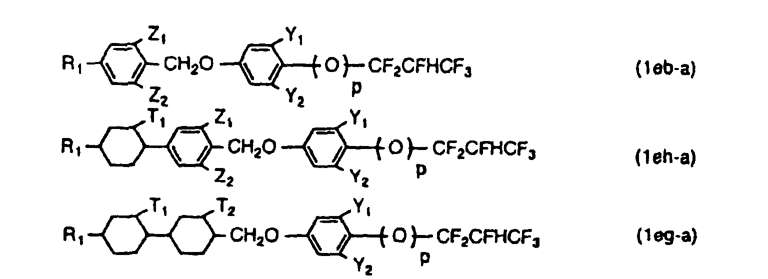

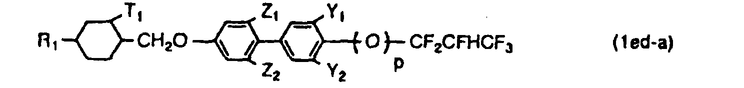

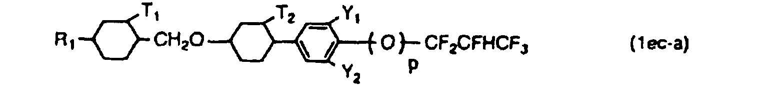

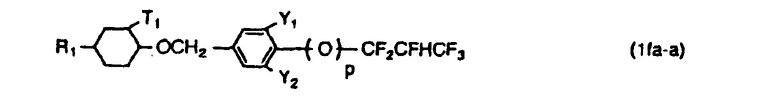

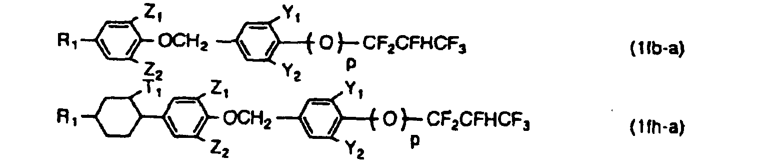

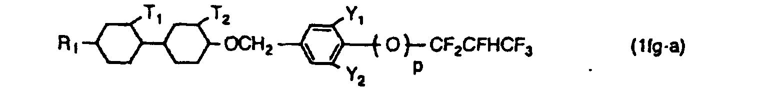

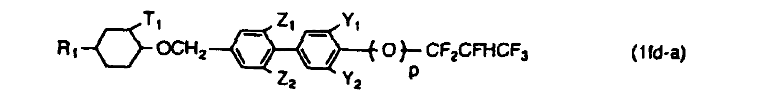

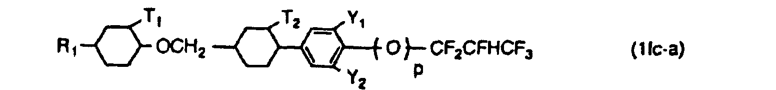

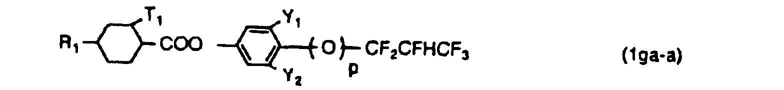

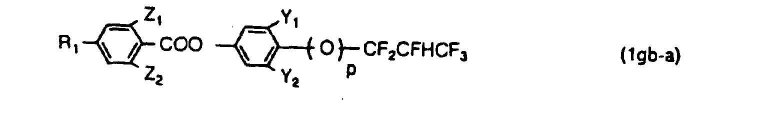

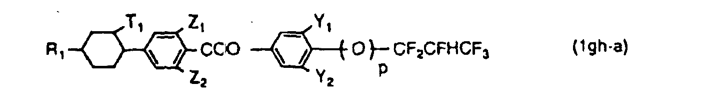

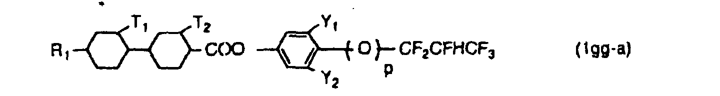

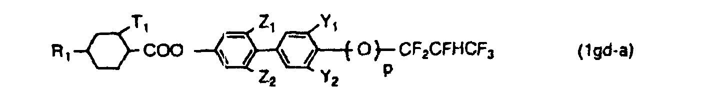

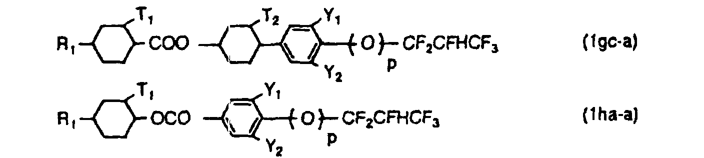

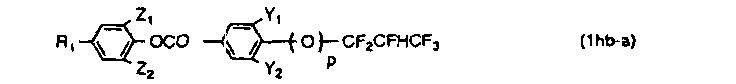

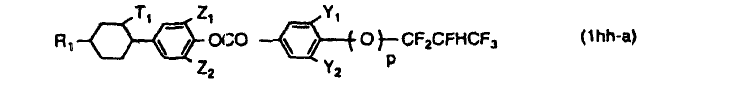

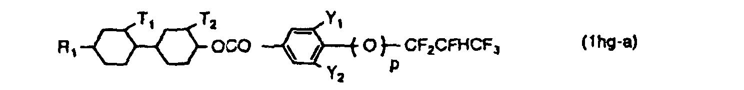

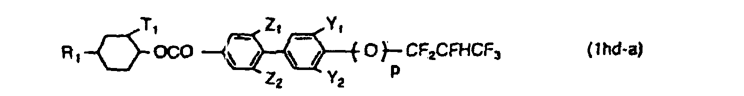

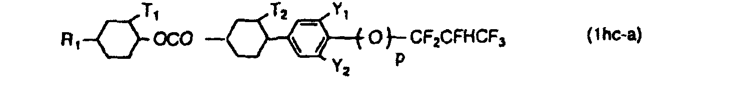

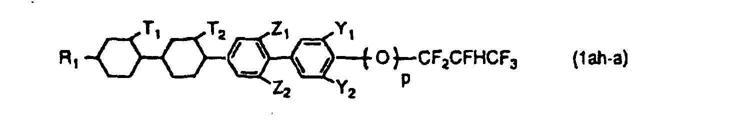

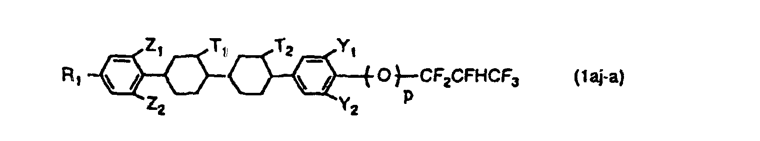

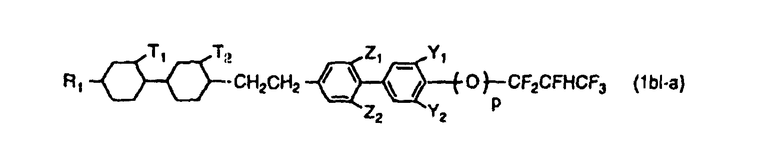

In these formulas, R₁, Y₁ and and Y₂ each are as defined above, and Z₁, Z₂ T₁ and T₂ each independently represent halogen atom or methyl group.

Any of the compounds expressed by the formula (1) can form the components constituting a liquid crystal composition having superior characteristics, and if a composition having a particularly high clearing point is required, it is preferred to select a tricyclic compound and a tetracyclic compound, while if a composition having a somewhat low clearing point is required, it is preferred to select a bicydic compound. 1,1,2,3,3,3-Hexafluoropropoxy group and 1,1,2,3,3,3-hexafluoropropyl group have a capability of exhibiting a large △ε, by themselves. However, if a larger Δε is required, it is preferred that at least one of Z₁, Z₂, Y₁, Y₂, T₁ and T₂ be halogen atom or X be -CO-O-. If a larger An is required, a compound containing a plurality of benzene rings or a compound of X = -C≡C- is preferred. Since the compound of the present invention has a core containing no group raising the viscosity, such as cyano group, any of the compounds have a low viscosity, but if a further lower viscosity is required, the problem can be solved by selecting a compound containing a plurality of hexane rings. As described above, when Z₁, Z₂, X, Y, T₁, T₂ and ring core are suitably selected, it is possible to optionally obtain compounds having required values of physical properties.

The compound expressed by the formula (1) of the present invention can be prepared according to the process expressed by the following equations, depending upon the kind of X:

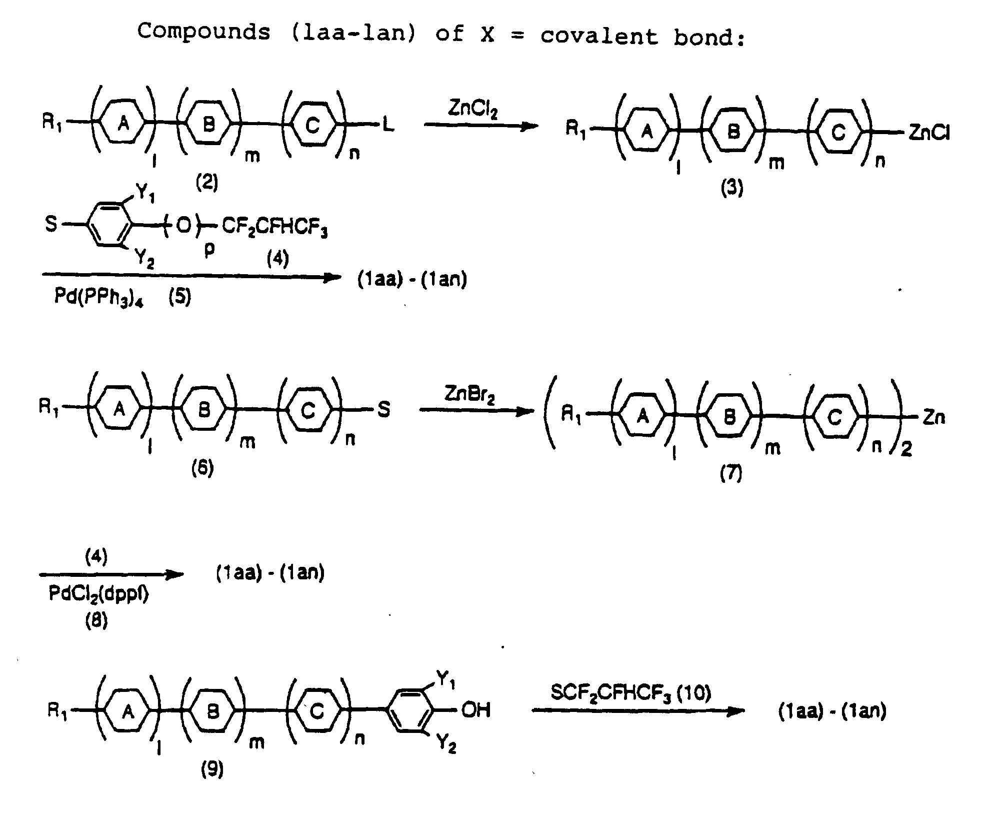

In the above equations, L represents a halogeno-magnesium or halogenolithium; S represents chlorine atom, bromine atom or iodine atom; Y, R₁, ℓ, m, n, p, rings A, B and C are as defined above.

These compounds can be prepared according to Negishi et al's method (Journal of the Organic Chemistry, 42, 1821 (1977), ditto 43, 358 (1978)). Namely, zinc chloride is reacted with a Grignard reagent or lithium compound (2) prepared from the corresponding halide in a conventional manner to obtain a zinc derivative (3), followed by reacting this compound with a 3,5-di-substituted-4-(1,1,2,3,3,3-hexafluoropropoxy)halogenobenzene, a 3-substituted-4-(1,1,2,3,3,3-hexafluoropropoxy)halogenobenzene, a 4-(1,1,2,3,3,3-hexafluoropropoxy)halogenobenzene, a 3,5-disubstituted-4-(1,1,2,3,3,3-hexafluoropropyl)halogenobenzene, a 3-substituted-4-(1,1,2, 3,3,3-hexafluoropropyl)halogenobenzene or a 4-(1,1,2,3,3,3-hexafluoropropyl)halogenobenzene (4), in the presence of a catalyst, to carry out cross-coupling reaction, thereby obtaining the compounds (1aa) to (1 an). As the catalyst used, a zero valence palladium catalyst is preferable.

Further, the compounds (1 aa) to (1 an) can also be prepared according to the method of Luche et al. (Journal of the Organic Chemistry, 48, 3837 (1983) or Tetrahedron Letters, 25, 3463 (1984)). Namely, zinc bromide is reacted with a halide (6) under irradiation of ultrasonic wave to obtain a zinc derivative (7), followed by reacting (4) with (7) in the presence of a catalyst to carry out a cross-coupling reaction, to obtain the compounds (1aa) to (1an). As the catalyst used, a divalent palladium catalyst is preferable.

Further, it is also possible to prepare the compounds by reacting 1,1,2,3,3,3-hexafluoropropyl iodide or 1,1,2,3,3,3-hexafluoropropyl bromide (10) with a phenol (9) under pressure. Further, the above Grignard reagent (2) is easily converted into a boric acid compound, and this compound can be derived into (1aa) to (1 an) according to Miller et al's method (Organometallics, 3, 1261 (1984)).

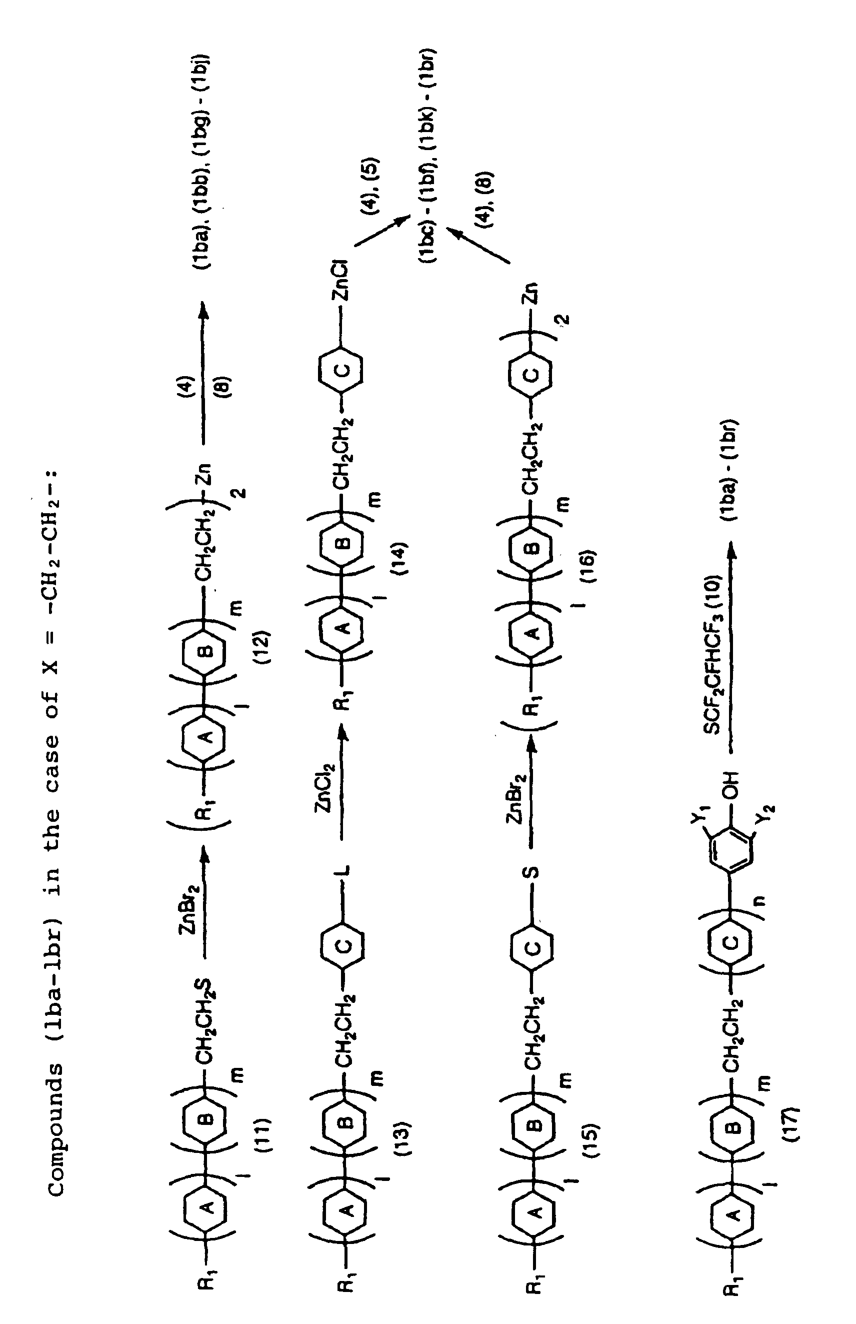

In the above equations, L, S, Y₁, Y₂, R₁, ℓ, m, n, p, rings A, B and C are as defined above.

In the case of n=0, zinc bromide is reacted with a halogen compound (11), as described above, followed by reacting a halogenobenzene (4) with the thus obtained zinc compound (12) in the presence of a catalyst, to prepare the compounds (1ba), (1bb), (1bg) to (1bj).

On the other hand, in the case of n=1, in the same manner as above, a Grignard reagent, a lithium compound (13) or a halide (15) is respectively converted into the corresponding zinc derivative (14) or (16), followed by reacting a halogenobenzene (4) in the presence of a catalyst (5) or (8), to prepare the compounds (1bc) to (1bf) and the compounds (1bk) to (1br). Further, even when a halide (10) is reacted with a phenol (17), it is possible to prepare the compounds (1ba) to (1br).

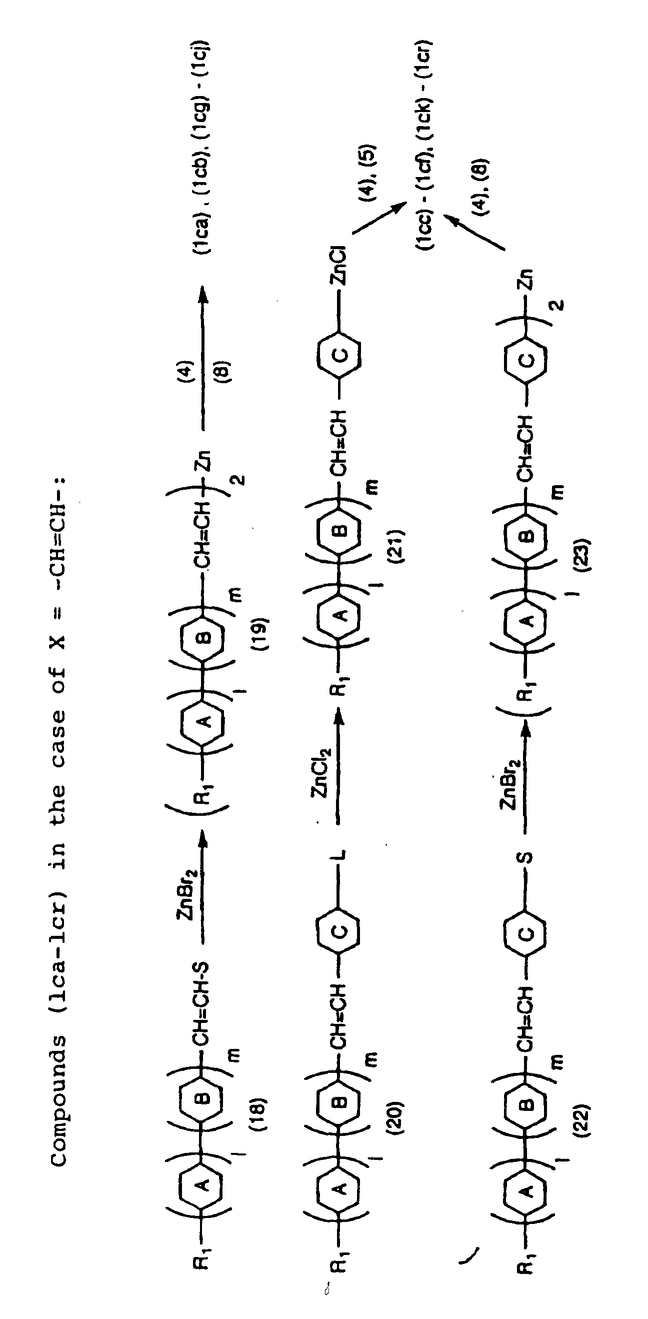

In the above equations, L, S, Y₁, Y₂, R₁, *ℓ,* m, n, p and rings A, B and C are as defined above.

In the case of n=0, in the same manner as above, zinc bromide is reacted with a halogen compound (18), followed by reacting a halogenobenzene (4) with the thus obtained zinc derivative (19) in the presence of a catalyst, to prepare compounds (1ca), (1cb), (1cg) to (1cj). On the other hand, in the case of n=1, in the same manner as above, a Grignard reagent, a lithium compound (20) or a halide (22) is respectively converted into the corresponding zinc derivatives (21) or (23), followed by reacting a halogenobenzene (4) in the presence of a catalyst (5) or (8), to obtain compounds (1cc) to (1cf) and (1ck) to (1cr).

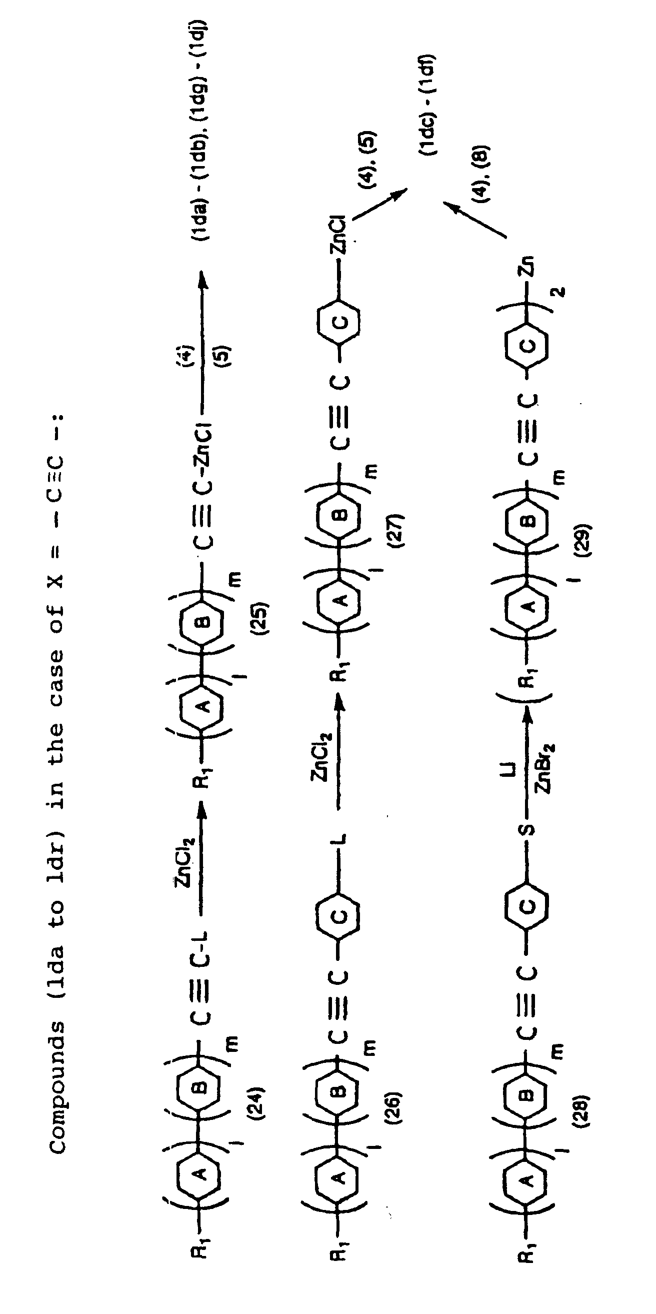

In the above equations, L, S, Y₁, Y₂, R₁, ℓ, m, n, p and rings A, B and C are as defined above.

In the case of n=0, in the same manner as above, zinc chloride is reacted with a Grignard reagent or a lithium compound (24), followed by reacting (4) with the thus obtained zinc derivative (25) in the presence of a catalyst (5), to obtain compounds (1da), (1db), (1dg) to (1dj). On the other hand, in the case of n=1, in the same manner as above, a Grignard reagent, a lithium compound (26) or a halide (28) is respectively converted into the corresponding zinc derivative (27) or (29), followed by reacting a halogenobenzene (4) in the presence of a catalyst (5) or (8), to prepare compounds (1dc) to (1df).

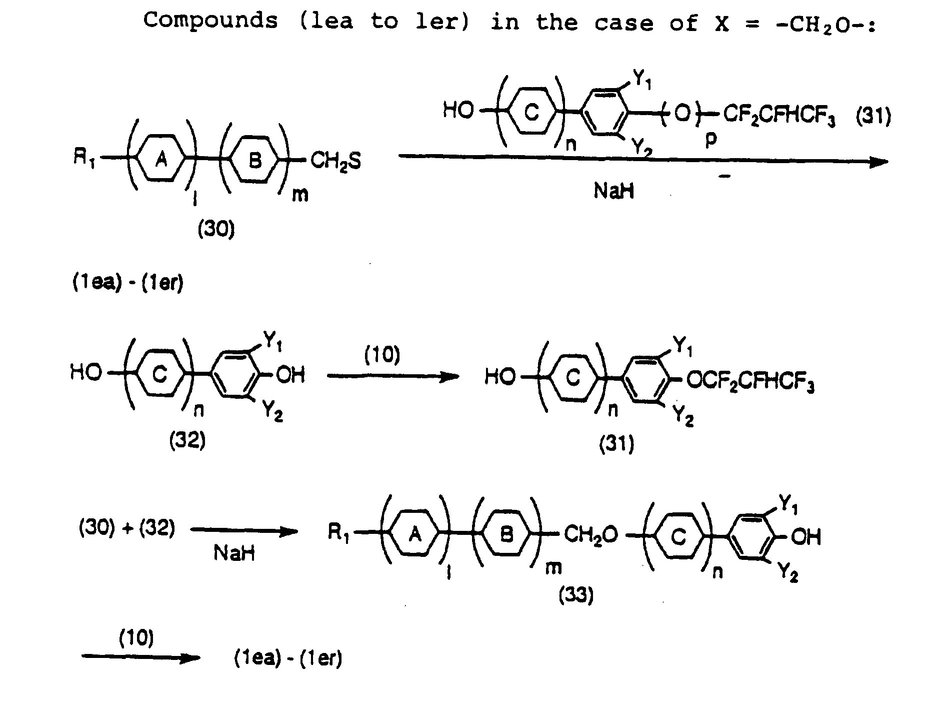

In the equations, S, Y₁, Y₂, R₁, ℓ, m, n, p and rings A, B and C are as defined above.

When sodium hydride and then a halide (30) are reacted with an alcohol or phenol (31), it is possible to prepare compounds (1ea) to (1er). In addition, (31) can be prepared by reacting a phenol (32) with a halide (10) in the same manner as above. Further, even when (30) is reacted with (32) and a halide (10) is reacted with the resulting phenol (33) in the same manner as above, compounds (1ea) to (1er) can be prepared.

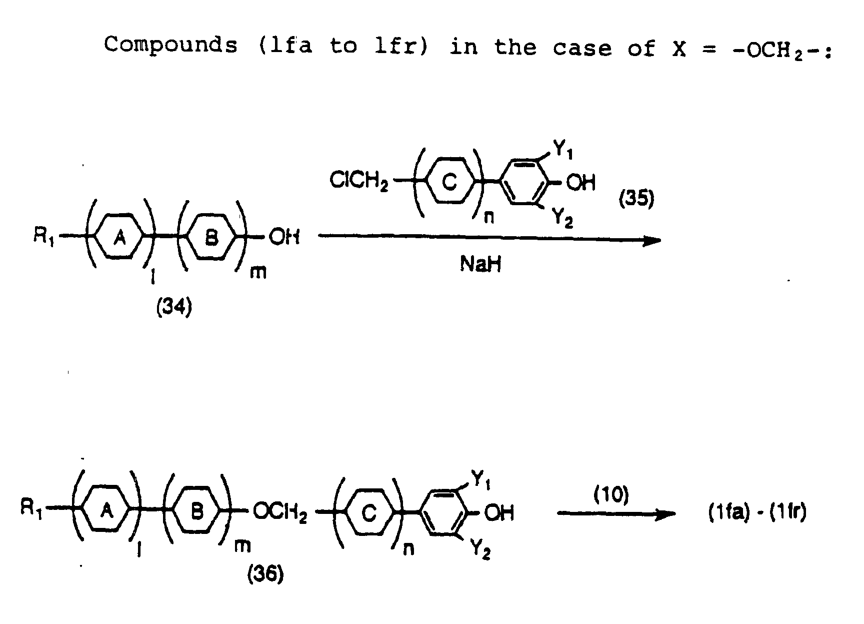

In the above equations, S, Y₁, Y₂, R₁, ℓ, m, n, p and rings A, B and C are as defined above.

When sodium hydride and then a chloride (35) are reacted with an alcohol or a phenol (34) and a halide (10) is reacted with the resulting phenol (36), compounds (1fa) to (1fr) can be prepared in the same manner as above. 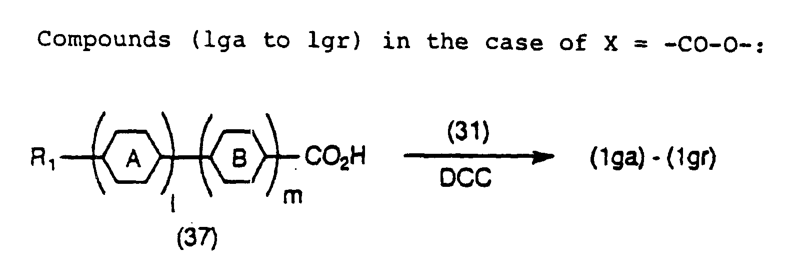 wherein R₁, ℓ, m and rings A and B are as defined above.

When a carboxylic acid (37) and an alcohol or a phenol (31) are subjected to dehydration-condensation, compounds (1ga) to (1gr) can be prepared.

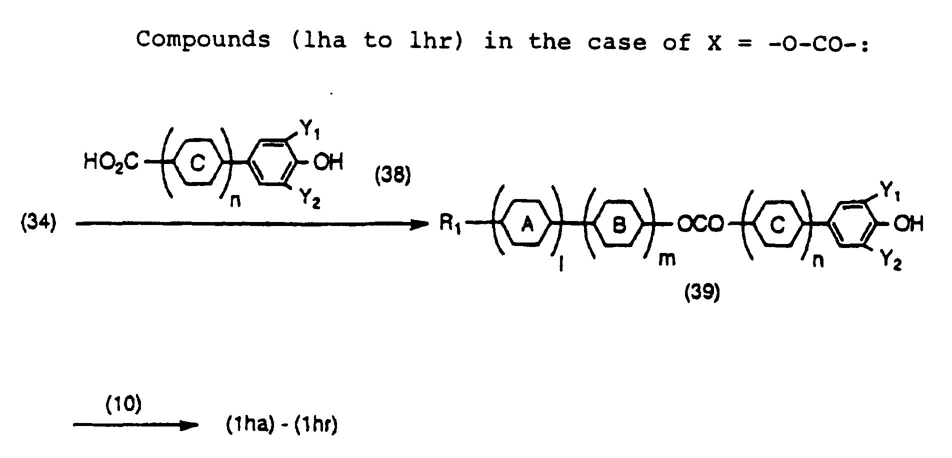

In the above equations, R₁, ℓ, m, n, p and rings A, B and C are as defined above.

When a carboxylic acid (38) and an alcohol or phenol (34) are subjected to dehydration-condensation, to obtain a phenol (39), followed by reacting a halide (10) with the phenol, it is possible to prepare compounds (1ha) to (1hr).

In addition, a compound of p=1 among compound (4) can be prepared from a 4-hydroxyhalogenobenzene and 1,1,2,3,3,3-hexafluoropropyl bromide or 1,1,2,3,3,3-hexafluoropropyl iodide (these propyl bromide or propyl iodide being collectively abbreviated to HFPs) or hexafluoropropene, according to the method described in Bulletine Society Chemistry of France, 923 (1974), for example. At that time, since HFPs as the raw material have a low boiling point, it is preferred to carry out the reaction in an autoclave under pressure in order to improve the reactivity. Further, a compound of p=0 among compound (4), can be prepared by reacting a HFPs with a phenyllithium derivative to carry out a coupling reaction.

The thus obtained compound (1) of the present invention is far superior as a constituting component of nematic liquid crystal compositions. Namely, since the compound easily mixes with various liquid crystal compounds, besides they have the above described superior characteristics, it is possible to provide nematic liquid crystal compositions suitable to electrooptical elements. Such liquid crystal compositions can be obtained preferably by blending a component (A) containing at least one kind of the compound (1) and besides, compounds optionally selected in accordance with objects, from a group of compounds (B) having a △ε ≧ 5, a group of compounds (C) having a |△ε|<5, a group of compounds (D) having particularly a clearing point of 80°C or higher and another group of compounds (E). As compounds included in (B), (C), (D) and (E), the following compounds can be preferably exemplified:

Namely, as compounds included in (B), the following (B1) to (B13) can be exemplified:

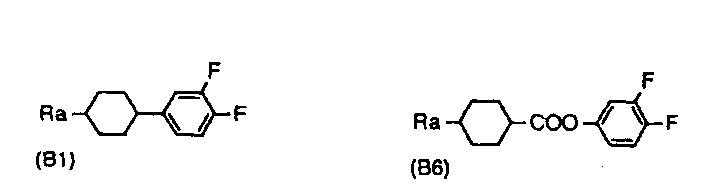

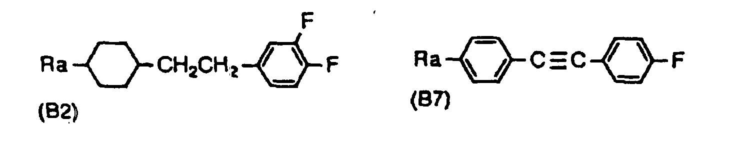

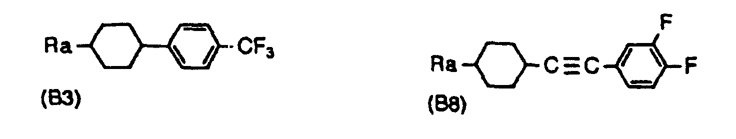

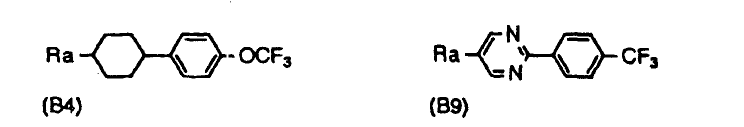

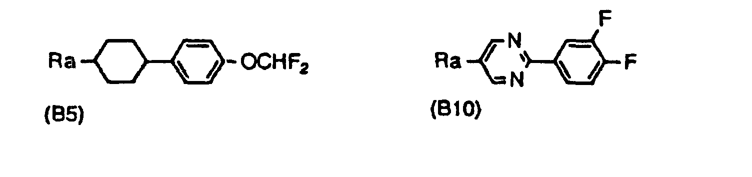

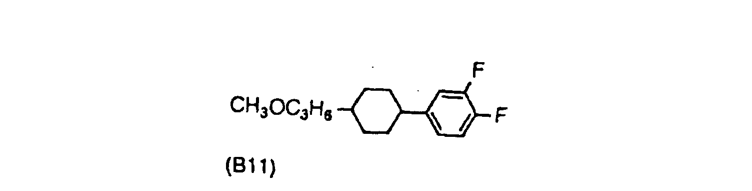

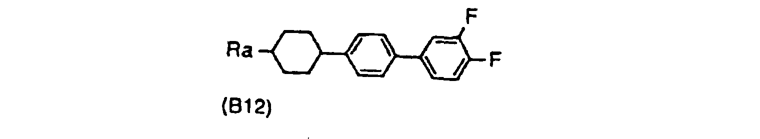

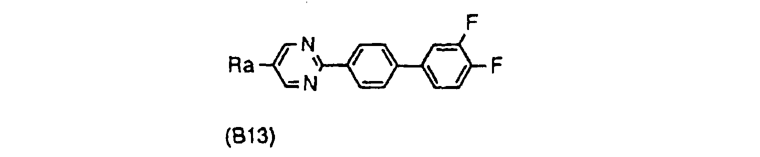

In the above formulas, Ra represents an alkyl group or alkenyl group having 1 to 10 carbon atoms, but one carbon atom or two or more not adjacent carbon atoms in the groups may be replaced by oxygen atom.

Further, as compounds corresponding to (C), the following (C1) to (C37) can be exemplified:

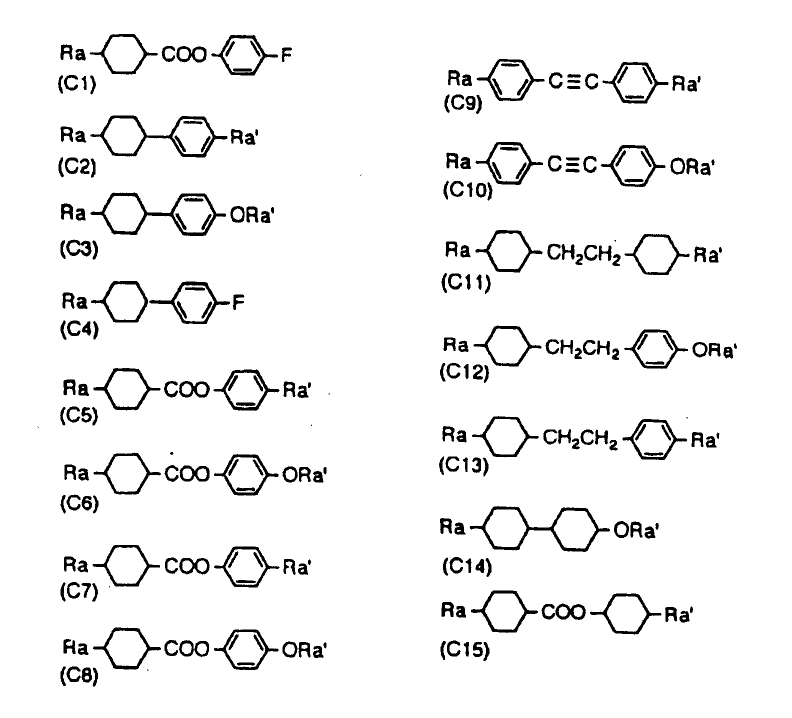

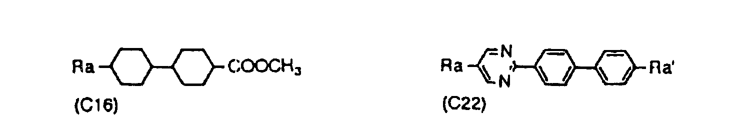

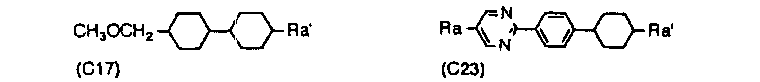

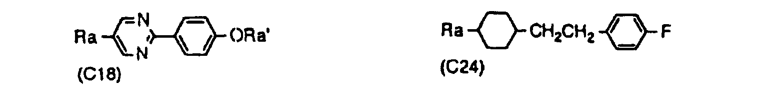

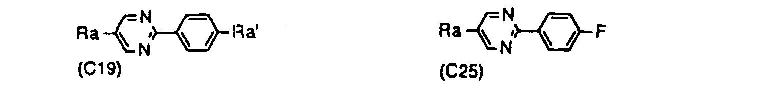

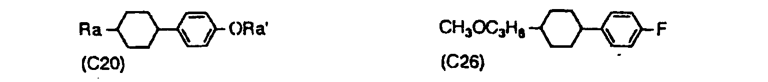

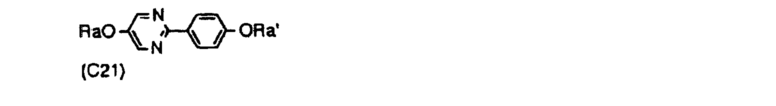

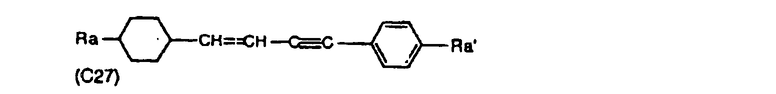

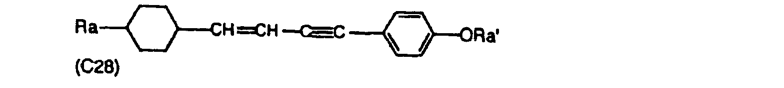

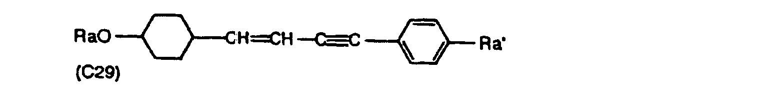

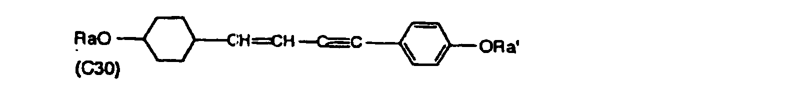

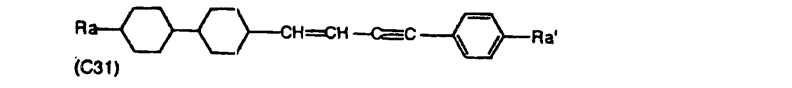

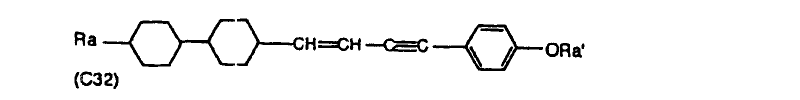

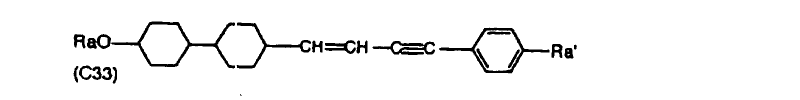

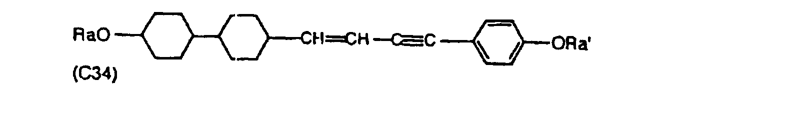

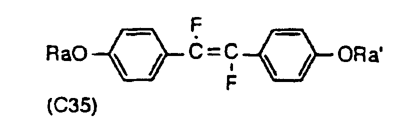

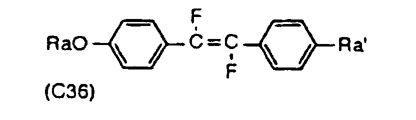

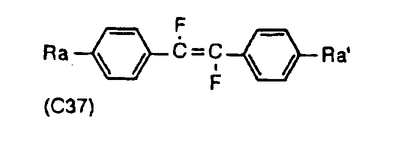

In the above formulas, Ra and Ra' each independently represent an alkyl group or alkenyl group having 1 to 10 carbon atoms, but one carbon atom or two or more not adjacent carbon atoms may be replaced by oxygen atom.

Further, as compounds included in (D), the following compounds (D1) to (D57) can be exemplified.

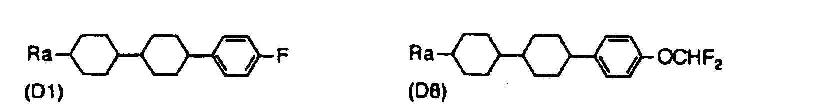

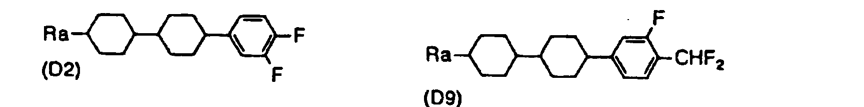

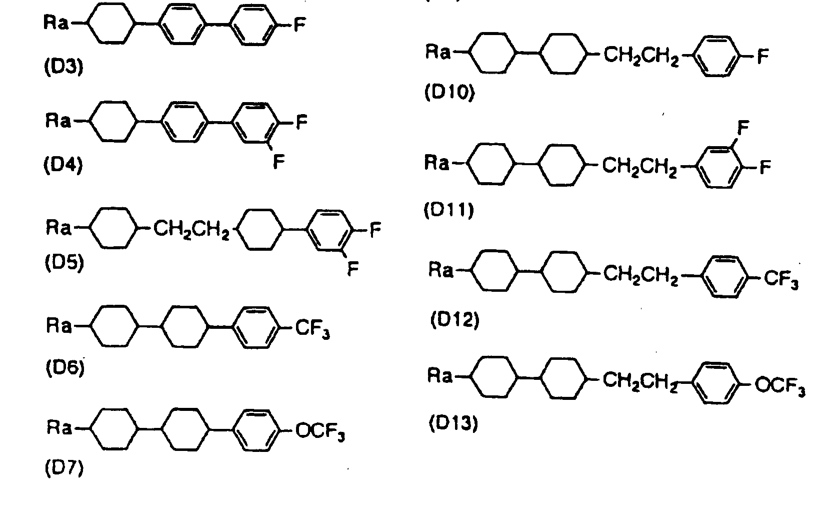

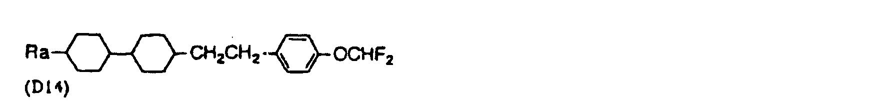

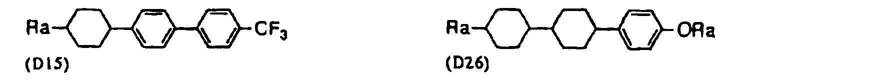

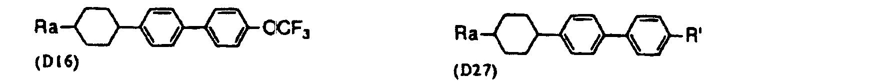

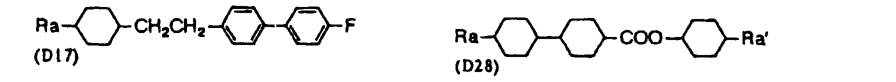

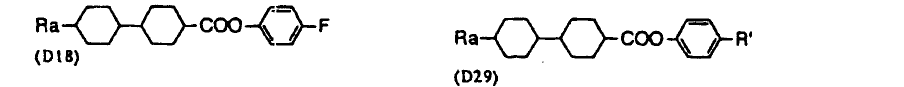

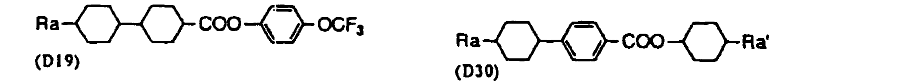

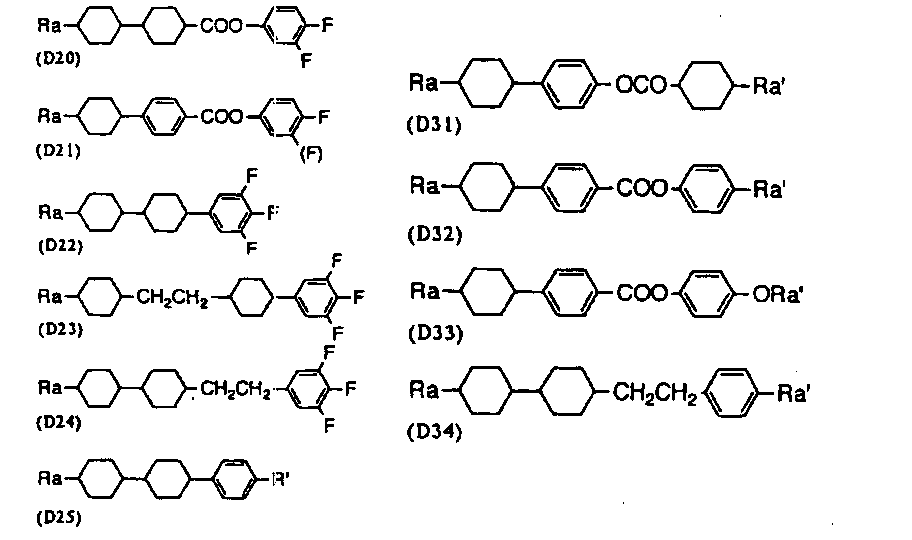

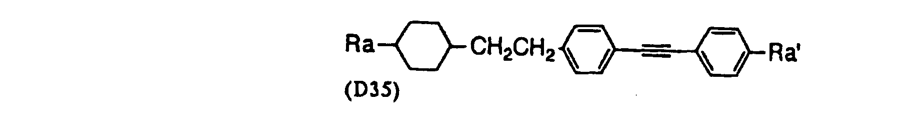

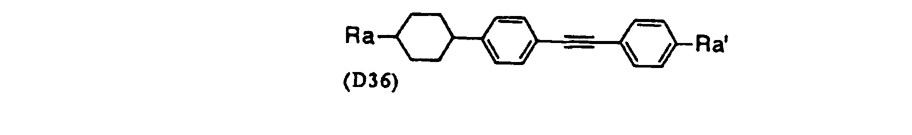

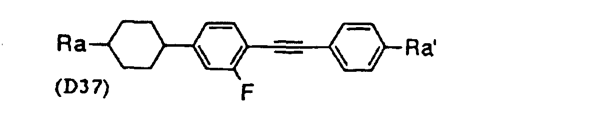

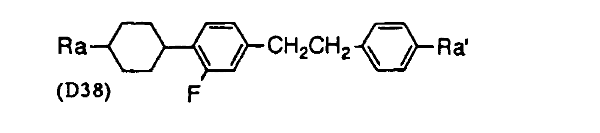

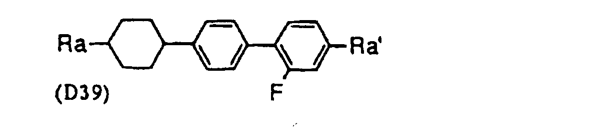

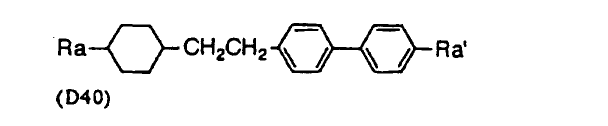

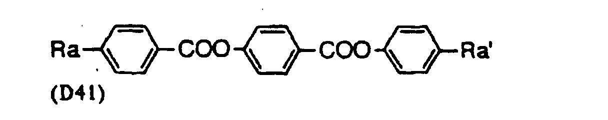

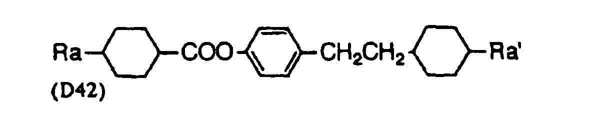

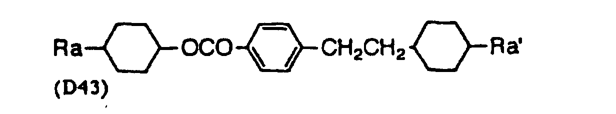

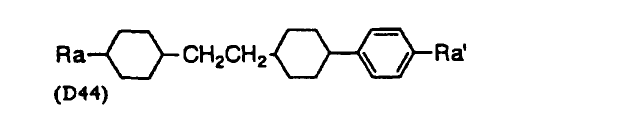

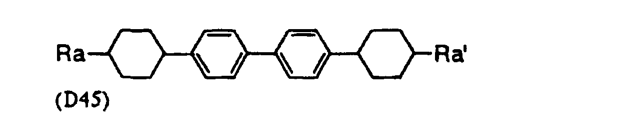

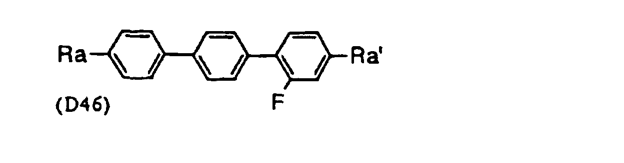

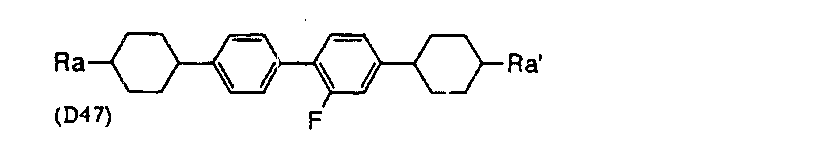

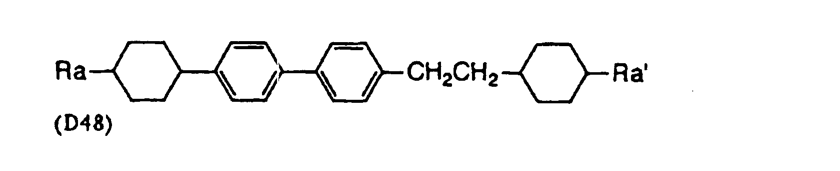

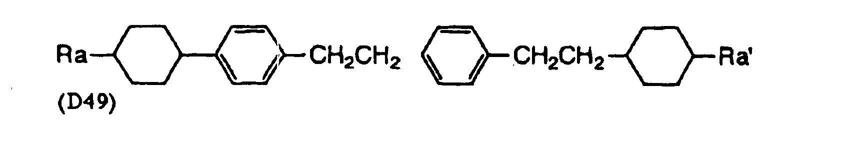

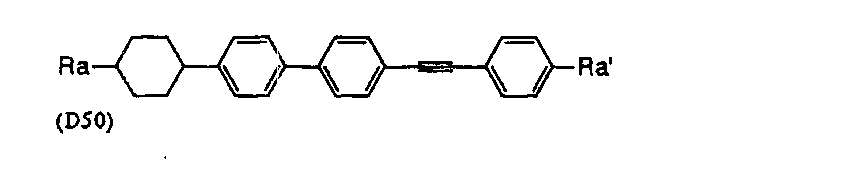

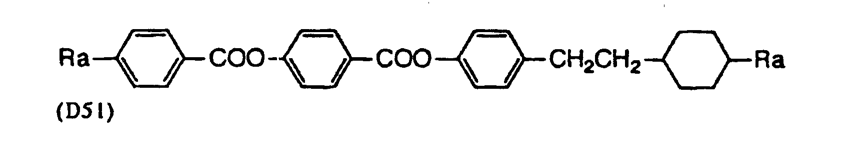

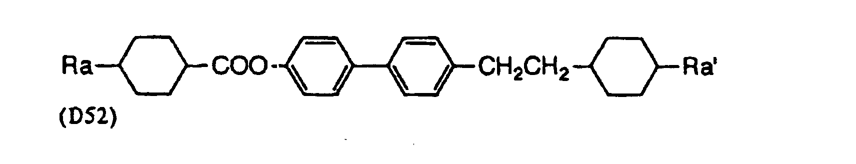

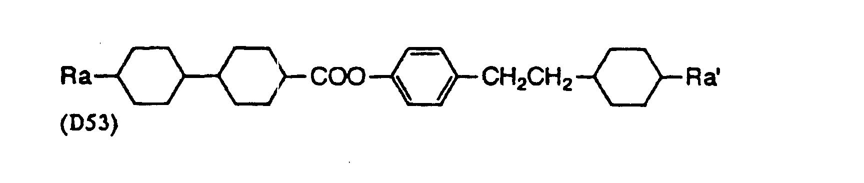

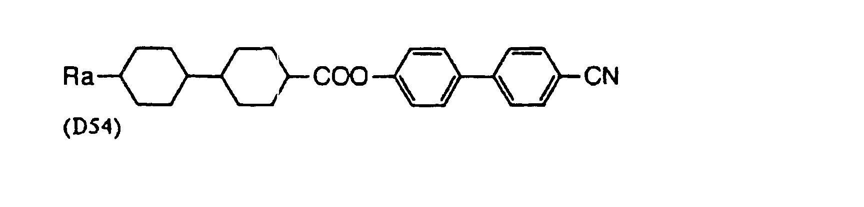

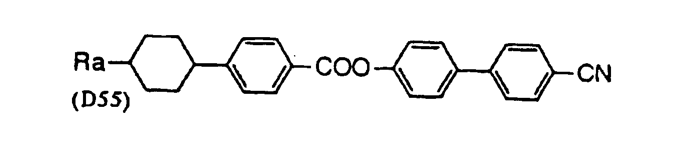

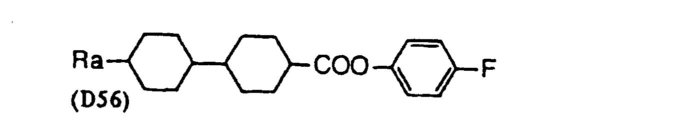

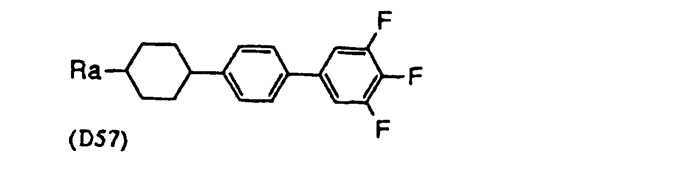

In the above formulas, Ra and Ra' are as defined above.
Further, as compounds included in (E), the following (E1) to (E22) can be exemplified.

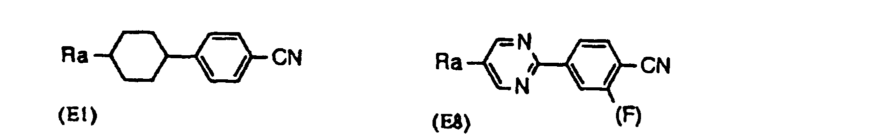

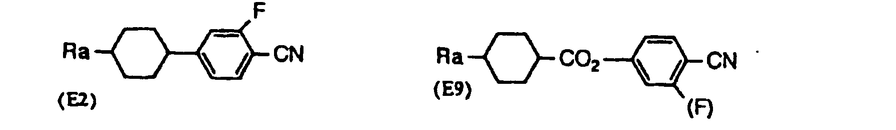

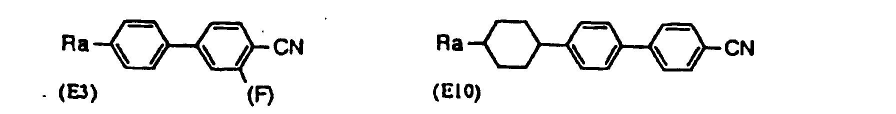

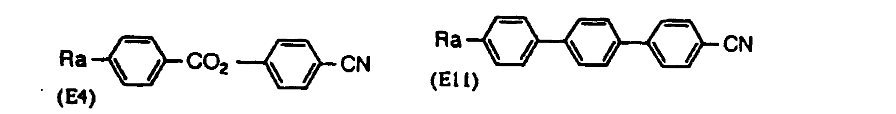

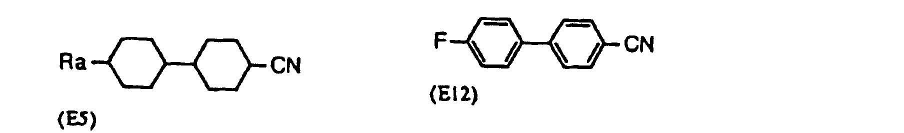

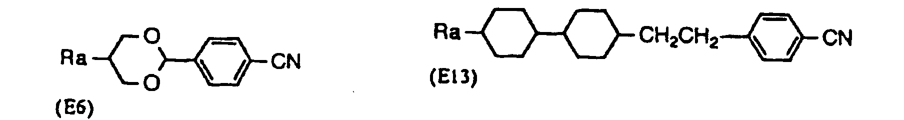

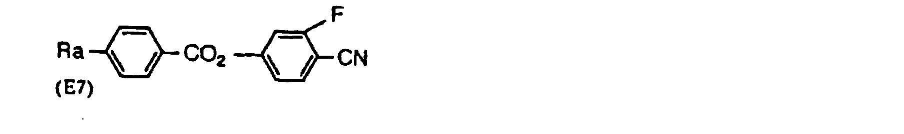

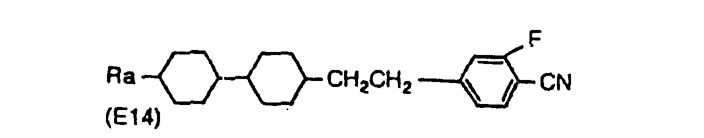

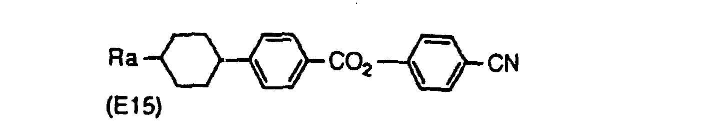

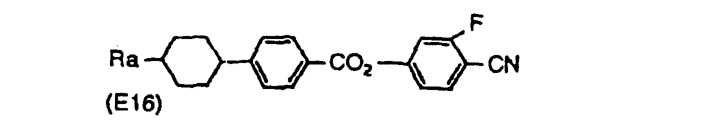

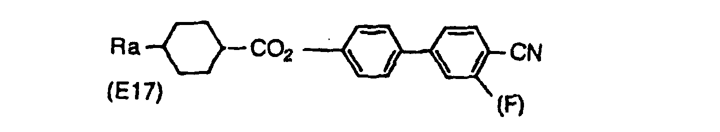

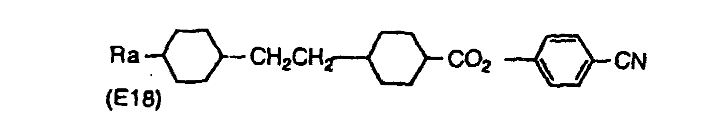

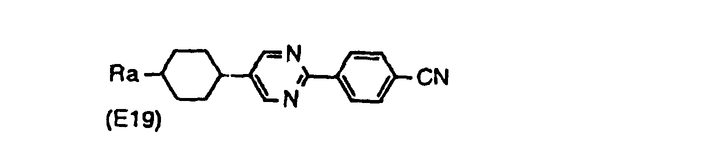

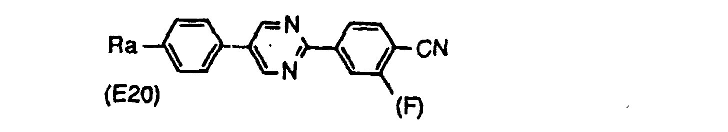

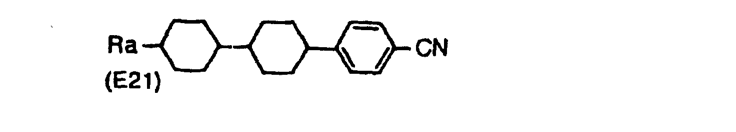

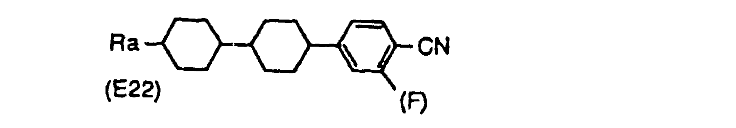

In the above formulas, Ra and Ra' are as defined above.

The liquid crystal composition of the present invention is preferred in that when one kind or more of compounds expressed by the formula (1) are contained therein in a proportion of 0.1 to 40% by weight, as the composition has superior liquid crystal characteristics.

Further, as nematic liquid crystal composition containing the compound of the present invention, composition examples mentioned below can be exemplified. In addition, compound Nos. are the same as those shown in Examples mentioned later. Hereinafter, 1,4-cyclohexylene and 1,2-ethenylene in side chains or central linkages refer to transconformation.

### Composition example 1

| | |
|---|---|
| 4'-(1,1,2,3,3,3-hexafluoropropoxy)-4-(4-propylcyclohexyl)biphenyl(compound No. 1) | 5% |
| 4'-(1,1,2,3,3,3-hexafluoropropoxy)-4-(4-pentylcyclohexyl)biphenyl(compound No. 17) | 5% |
| 2-fluoro-4-(4-(4-propylcyclohexyl)cyclohexyl)-1-(1,1,2,3,3,3-hexafluoropropoxy)benzene | 5% |
| 2-fluoro-4-(4-(4-pentylcyclohexyl)cyclohexyl)-1-(1,1,2,3,3,3-hexafluoropropoxy)benzene | 5% |
| 1,2,3-trifluoro-5-(4-heptylcyclohexyl)benzene | 9% |
| 1,2-difluoro-4-(4-(4-ethylcyclohexyl)cyclohexyl)benzene | 5% |
| 1,2-difluoro-4-(4-(4-propylcyclohexyl)cyclohexyl)benzene | 5% |
| 1,2-difluoro-4-(4-(4-pentylcyclohexyl)cyclohexyl)benzene | 5% |
| 1,2,3-trifluoro-5-(4-(2-(4-propylcyclohexyl)ethyl)cyclohexyl)benzene | 7% |
| 1,2,3-trifluoro-5-(4-(2-(4-butylcyclohexyl)ethyl)cyclohexyl)benzene | 7% |
| 1,2,3-trifluoro-5-(4-(2-(4-pentylcyclohexyl)ethyl)cyclohexyl)benzene | 6% |
| 1,2,3-trifluoro-5-(4-(4-propylcyclohexyl)cyclohexyl)benzene | 10% |
| 1,2,3-trifluoro-5-(4-(4-butylcyclohexyl)cyclohexyl)benzene | 5% |
| 1,2,3-trifluoro-5-(2-(4-(4-propylcyclohexyl)cyclohexyl)ethyl)benzene | 11% |
| 1,2,3-trifluoro-5-(2-(4-(4-pentylcyclohexyl)cyclohexyl)ethyl)benzene | 10% |

### Composition example 2

| | |
|---|---|
| 2',6'-difluoro-4'-(4-propylcyclohexyl)-4-(1,1,2,3,3,3-hexafluoropropoxy)biphenyl(compound No. 47) | 5% |
| 2',6'-difluoro-4'-(4-pentylcyclohexyl)-4-(1,1,2,3,3,3-hexafluoropropoxy)biphenyl(compound No. 49) | 5% |
| 2',6'-difluoro-4-(4-(4-propylcyclohexyl)cyclohexyl)-1-(1,1,2,3,3,3-hexafluoropropoxy)benzene | 5% |
| 2',6'-difluoro-4-(4-(4-pentylcyclohexyl)cyclohexyl)-1-(1,1,2,3,3,3-hexafluoropropoxy)benzene | 5% |
| 4-(4-propylcyclohexyl)-1-(1,1,2,3,3,3-hexafluoropropoxy)benzene(compound No. 91) | 5% |
| 4-(4-pentylcyclohexyl)-1-(1,1,2,3,3,3-hexafluoropropoxy)benzene(compound No. 98) | 5% |
| 1,2,3-trifluoro-5-(4-heptylcyclohexyl)benzene | 6% |
| 1,2,3-trifluoro-5-(4-(2-(4-propylcyclohexyl)ethyl)cyclohexyl)benzene | 10% |
| 1,2,3-trifluoro-5-(4-(2-(4-butylcyclohexyl)ethyl)cyclohexyl)benzene | 9% |
| 1,2,3-trifluoro-5-(4-(2-(4-pentylcyclohexyl)ethyl)cyclohexyl)benzene | 10% |
| 1,2,3-trifluoro-5-(4-(4-propylcyclohexyl)cyclohexyl-benzene | 10% |
| 1,2,3-trifluoro-5-(2-(4-(4-propylcyclohexyl)cyclohexyl)ethyl)benzene | 5% |
| 1,2,3-trifluoro-5-(2-(4-(4-pentylcyclohexyl)cyclohexyl)ethylbenzene | 5% |
| 3,4,5-trifluoro-4'-(4-propylcyclohexyl)bipheny) | 10% |
| 3,4,5-trifluoro-4'-(4-pentylcyclohexyl)biphenyl | 5% |

### Composition example 3

| | |
|---|---|
| 4-propylcyclohexyl-1-(1,1,2,3,3,3-hexafluoropropyl)benzene(compound No. 92) | 10% |
| 4-(4-(2-(4-propylcyclohexyl)ethyl)cyclohexyl)-1-(1,1,2,3,3,3-hexafluoropropyl)benzene (compound No. 200) | 10% |
| 4-(4-(2-(4-pentylcyclohexyl)ethyl)cyclohexyl)-1-(1,1,2,3,3,3-hexafluoropropyl)benzene (compound No. 202) | 10% |
| 1,2,3-trifluoro-5-(4-(4-propylcyclohexyl)cyclohexyl-benzene | 10% |
| 1,2,3-trifluoro-5-(4-(4-butylcyclohexyl)cyclohexyl)benzene | 5% |
| 1,2,3-trifluoro-5-(2-(4-(4-propylcyclohexyl)ethyl)cyclohexyl)benzene | 10% |
| 1,2,3-trifluoro-5-(2-(4-(4-pentylcyclohexyl)ethyl)cyclohexyl)benzene | 10% |
| 1,2,3-trifluoro-5-(2-(4-(4-propylcyclohexyl)cyclohexyl)ethyl)benzene | 10% |
| 1,2,3-trifluoro-5-(2-(4-(4-pentylcyclohexyl)cyclohexyl)ethyl)benzene | 10% |
| 3,4,5-trifluoro-4'-(4-propylcyclohexyl)biphenyl | 8% |
| 3,4,5-trifluoro-4'-(4-pentylcyclohexyl)biphenyl | 7% |

### Composition example 4

| | |
|---|---|
| 4'-(4-propylcyclohexyl)-3,5-difluoro-4-(1,1,2,3,3,3-hexafluoropropoxy)biphenyl | 5% |
| 4'-(4-pentylcyclohexyl)-3,5-difluoro-4-(1,1,2,3,3,3-hexafluoropropoxy)biphenyl | 5% |
| 4'-(2-(4-propylcyclohexyl)ethyl)-4-(1,1,2,3,3,3-hexafluoropropoxy)biphenyl(compound No. 191) | 5% |
| 2,6-difluoro-4-(4-propylcyclohexyl)-1-(1,1,2,3,3,3-hexafluoropropoxy)benzene | 5% |
| 2,6-difluoro-4-(4-pentylcyclohexyl)-1-(1,1,2,3,3,3-hexafluoropropoxy)benzene | 5% |
| 1,2,3-trifluoro-5-(2-(4-pentylcyclohexyl)ethyl)benzene | 4% |
| 4-fluorophenyl 4-pentylcyclohexanecarboxylate | 9% |
| 4-fluorophenyl 4-heptylcyclohexanecarboxylate | 9% |
| 1-methyl-4-(4-(4-propylcyclohexyl)cyclohexyl)benzene | 6% |
| 1,2-difluoro-4-(4-(4-ethylcyclohexyl)cyclohexyl)benzene | 8% |
| 1,2-difluoro-4-(4-(4-propylcyclohexyl)cyclohexyl)benzene | 8% |
| 1,2-difluoro-4-(4-(4-pentylcyclohexyl)cyclohexyl)benzene | 7% |
| 1,2-difluoro-4'-(4-ethylcyclohexyl)biphenyl | 6% |
| 1,2-difluoro-4'-(4-propylcyclohexyl)biphenyl | 6% |
| 1,2-difluoro-4'-(4-pentylcyclohexyl)biphenyl | 12% |

### Composition example 5

| | |
|---|---|
| 4'-(4-propylcyclohexyl)-3-fluoro-4-(1,1,2,3,3,3-hexafluoropropoxy)biphenyl | 5% |
| 4'-(4-pentylcyclohexyl)-3-fluoro-4-(1,1,2,3,3,3-hexafluoropropoxy)biphenyl | 5% |
| 4-(4-propylcyclohexyl)cyclohexyl-1-(1,1,2,3,3,3-hexafluoropropoxy)benzene(compound No. 110) | 5% |
| 4-(4-(4-pentylcyclohexyl)cyclohexyl-1-(1,1,2,3,3,3-hexafluoropropoxy)benzene(compound No. 111) | 5% |
| 2-fluoro-4-(4-propylcyclohexyl)-1-(1,1,2,3,3,3-hexafluoropropoxy)benzene | 5% |
| 2-fluoro-4-(4-pentylcyclohexyl)-1-(1,1,2,3,3,3-hexafluoropropoxy)benzene | 5% |
| 1,2-difluoro-4-(4-(4-ethylcyclohexyl)cyclohexyl)benzene | 10% |
| 1,2-difluoro-4-(4-(4-propylcyclohexyl)cyclohexyl)benzene | 10% |
| 1,2-difluoro-4-(4-(4-pentylcyclohexyl)cyclohexyl)benzene | 10% |
| 1,2,3-trifluoro-5-(4.(4-ethylcyclohexyl)cyclohexyl)benzene | 10% |
| 1,2,3-trifluoro-5-(4.(4-pentylcyclohexyl)cyclohexyl)benzene | 10% |
| 1,2,3-trifluoro-5-(4-(2-(4-ethylcyclohexyl)ethyl)cyclohexyl)benzene | 10% |
| 1,2,3-trifluoro-5-(2-(4-(4-propylcyclohexyl)cyclohexyl)ethyl)benzene | 10% |

### Composition example 6

| | |
|---|---|
| 2'-fluoro-4'-(4-propylcyclohexyl)-4-1,1,2,3,3,3-hexafluoropropoxy)biphenyl(compound No. 34) | 5% |
| 2'-fluoro-4'-(4-pentylcyclohexyl)-4-(1,1,2,3,3,3-hexafluoropropoxy)biphenyl(compound No. 36) | 5% |
| 4'-(4-propylcyclohexyl)-3,5-difluoro-4-(1,1,2,3,3,3-hexafluoropropoxy)biphenyl(compound No. 28) | 5% |
| 4'-(4-pentylcyclohexyl)-3,5-difluoro-4-(1,1,2,3,3,3-hexafluoropropoxy)biphenyl(compound No. 29) | 5% |
| 1-chloro-4-(4-propylcyclohexyl)benzene | 5% |
| 1-chloro-4-(4-pentylcyclohexyl)benzene | 5% |
| 1-chloro-4-(4-heptylcyclohexyl)benzene | 5% |
| 1,2-difluoro-4-(4-(2-(4-ethylcyclohexyl)ethyl)cyclohexyl)benzene | 12% |
| 1,2-difluoro-4-(4-(2-(4-propylcyclohexyl)ethyl)cyclohexyl)benzene | 6% |
| 1,2-difluoro-4-(4-(2-(4-pentylcyclohexyl)ethyl)cyclohexyl)benzene | 12% |
| 1-chloro-4-(4-(4-ethylcyclohexyl)cyclohexyl)benzene | 5% |
| 1-chloro-4-(4-(4-propylcyclohexyl)cyclohexyl)benzene | 5% |
| 1-chloro-4-(4-(4-butylcyclohexyl)cyclohexyl)benzene | 5% |
| 1-chloro-4-(4-(4-pentylcyclohexyl)cyclohexyl)benzene | 5% |
| 1-chloro-2-fluoro-4-(4-(2-(4-ethylcyclohexyl)ethyl)cyclohexyl)benzene | 6% |
| 1-chloro-2-fluoro-4-(4-(2-(4-propylcyclohexyl)ethyl)cyclohexyl)benzene | 5% |
| 1-chloro-2-fluoro-4-(4-(2-(4-butylcyclohexyl)ethyl)cyclohexyl)benzene | 5% |

### Composition example 7 (for comparison purposes)

| | |
|---|---|
| 2-fluoro-4-(2-(4-(4-ethylcyclohexyl)phenyl)ethynyl)-1-(1,1,2,3,3,3-hexafluoropropoxy)benzene | 5% |
| 2-fluoro-4-(2-(4-(4-propylcyclohexyl)phenyl)ethynyl)-1-(1,1,2,3,3,3-hexafluoropropoxy)benzene | 5% |
| 2-fluoro-4-(2-(4-(4-pentylcyclohexyl)phenyl)ethynyl)-1-(1,1,2,3,3,3-hexafluoropropoxy)benzene | 5% |
| 4'-ethyl-4-cyanobiphenyl | 7% |
| 4'-butyl-4-cyanobiphenyl | 4% |
| 1-cyano-4-(4-ethylcyclohexyl)benzene | 12% |
| 1-cyano-4-(4-propylcyclohexyl)benzene | 3% |
| 2-(3,4-difluorophenyl)-5-propylpyrimidine | 6% |
| 1-propyl-4-(4-methoxymethylcyclohexyl)cyclohexane | 6% |
| 1-pentyl-4-(4-methoxymethylcyclohexyl)cyclohexane | 5% |
| 1-methyl-4-(4-(4-ethylcyclohexyl)cyclohexyl)benzene | 4% |
| 1-methyl-4-(4-(4-propylcyclohexyl)cyclohexyl)benzene | 5% |
| 1-fluoro-4-(4-(4-propylcyclohexyl)cydohexyl)benzene | 4% |
| 1-methoxy-4-(4-(4-propylcyclohexyl)cyclohexyl)benzene | 4% |
| 1-propyl-4-(4-(4-propylcyclohexyl)cyclohexyl)benzene | 11% |
| 1-cyano-4-(4-(4-propylcyclohexyl)cyclohexyl)benzene | 8% |
| 4-fluorophenyl 4-(4-propylcyclohexyl)benzoate | 6% |

### Composition example 8

| | |
|---|---|
| 4-(2-(4-(4-ethylcyclohexyl)phenyl)ethynyl)-1-(1,1,2,3,3,3-hexafluoropropoxy)benzene (compound No. 267) | 5% |
| 4-(2-(4-(4-propylcyclohexyl)phenyl)ethynyl)-1-(1,1,2,3,3,3-hexafluoropropoxy)benzene (compound No. 283) | 5% |
| 4-(2-(4-(4-pentylcyclohexyl)phenyl)ethynyl)-1-(1,1,2,3,3,3-hexafluoropropoxy)benzene (compound No. 285) | 5% |
| 2-(3,4-difluorophenyl)-5-pentylpyrimidine | 13% |
| 1-cyano-2-fluoro-4-ethylcyclohexyl)benzene | 5% |
| 1-cyano-2-fluoro-4-(4-propylcyclohexyl)benzene | 8% |
| 4'-ethyl-4-cyanobiphenyl | 5% |
| 1-cyano-4-(4-propylcyclohexyl)benzene | 2% |
| 1-cyano-4-(4-(4-ethylcyclohexyl)cyclohexyl)benzene | 6% |
| 1-cyano-4-(4-(4-propylcyclohexyl)cyclohexyl)benzene | 6% |
| 1-cyano-4-(4-(4-butylcyclohexyl)cyclohexyl)benzene | 6% |
| 1-cyano-4-(4-(4-pentylcyclohexyl)cyclohexyl)benzene | 6% |
| 1-methyl-4-(4(-4-propylcyclohexyl)cyclohexyl)benzene | 5% |
| 1-cyano-2-fluoro-4-(4-(4-ethylcyclohexyl)cyclohexyl)benzene | 6% |
| 1-cyano-2-fluoro-4-(4-(4-propylcyclohexyl)cyclohexyl)benzene | 6% |
| 2-(4'-fluorobiphenyl)-5-propylpyrimidine | 5% |
| 2-(4'-fluorobiphenylyl)-5-butylpyrimidine | 5% |

### Composition example 9

| | |
|---|---|
| 2,6-difluoro-4-(2-(4-(4-propylcyclohexyl)phenyl)ethynyl)-1-(1,1,2,3,3,3-hexafluoropropoxy)benzene | 5% |
| 2,6-difluoro-4-(2-(4-4-pentylcycloheyl)phenyl)ethynyl)-1-(1,1,2,3,3,3-hexafluoropropoxy)benzene | 5% |
| 4-(2-(4-(4-propylcyclohexyl)phenyl)ethynyl)-1-(1,1,2,3,3,3-hexafluoropropoxy)benzene (compound No. 283) | 5% |
| 1-cyano-4-(4-(3-butenyl)cyclohexyl)benzene | 8% |
| 1-cyano-4-(4-(3-pentenyl)cyclohexyl)benzene | 7% |
| 1-cyano-4-(4-propylcyclohexyl)benzene | 7% |
| 1-methyl-4-(2-(4-ethylphenyl)ethynyl)benzene | 5% |
| 1-methyl-4-(2-(4-hexylphenyl)ethynyl)benzene | 10% |
| 1-butyl-4-(2-(4-butylphenyl)ethynyl)benzene | 5% |
| 1-methoxy-4-(2-(4-ethylphenyl)ethynyl)benzene | 11% |
| 2-(4'-fluorobiphenylyl)-5-propylpyrimidine | 6% |
| 2-(4'-fluorobiphenylyl)-5-butylpyrimidine | 6% |
| 1-ethyl-4-(2-(2-fluoro-4-(4-propylcyclohexyl)phenyl)ethynyl)benzene | 4% |
| 1-propyl-4-(2-(2-fluoro-4-(4-propylcyclohexyl)phenyl)ethynyl)benzene | 4% |
| 1-ethyl-4-(2-(4-(2-(4-propylcyclohexyl)ethyl)phenyl)ethynyl)benzene | 4% |
| 1-propyl-4-(2-(4-(2-(4-propylcyclohexyl)ethyl)phenyl)ethynyl)benzene | 4% |
| 1-butyl-4-(2-(4-(2-(4-propylcyclohexyl)ethyl)phenyl)ethynyl)benzene | 4% |

### Composition example 10

| | |
|---|---|
| 2-fluoro-4-(2-(4-(4-pentylcyclohexyl)phenyl)ethynyl)-1-(1,1,2,3,3,3-hexafluoropropoxy)benzene | 5% |
| 4-(2-(4-(4-ethylcyclohexyl)phenyl)ethynyl)-1-(1,1,2,3,3,3-hexafluoropropoxy)benzene (compound No. 267) | 5% |
| 4-(2-(4-(4-propylcyclohexyl)phenyl)ethynyl)-1-(1,1,2,3,3,3-hexafluoropropoxy)benzne (compound No. 283) | 5% |
| 4'-ethyl-4-cyanobiphenyl | 7% |
| 4'-pentyl-4-cyanobiphenyl | 8% |
| 1-cyano-4-(4-methoxymethylcyclohexyl)benzene | 8% |
| 1-cyano-4-(4-propylcyclohexyl)benzene | 8% |
| 1-pentyl-4-(4-methoxymethylcyclohexyl)cyclohexane | 4% |
| 2-(4-ethylphenyl)-5-ethylpyrimidine | 3% |
| 2-(4-ethylphenyl)-5-propylpyrimidine | 3% |
| 2-(4-ethylphenyl)-5-butylpyrimidine | 3% |
| 2-(4-(4-propylcyclohexyl)phenyl)-5-ethylpyrimidine | 6% |
| 2-(4-(4-propylcyclohexyl)phneyl)-5-propylpyrimidine | 6% |
| 1-methyl-4-(4-(4-propylcyclohexyl)cyclohexyl]benzene | 7% |
| 1-methoxy-4-(4-(4-propylcyclohexyl)cyclohexyl)benzne | 4% |
| 1-fluoro-4-(4-(4-propylcyclohexyl)cyclohexyl)benzene | 3% |
| 1.2-difluoro-4-(4-(4-ethylcyclohexyl)cyclohexyl)benzene | 5% |
| 1,2-difluoro-4-(4-(4-propylcyclohexyl)cyclohexyl)benzene | 5% |
| 1,2-difluoro-4-(4-(4-pentylcyclohexyl)cyclohexyl)benzene | 5% |

### Composition example 11 (for comparison purposes)

| | |
|---|---|
| 2-fluoro-4-(2-(4-(4-ethylcyclohexyl)phenyl)ethynyl)-1-(1,1,2,3,3,3-hexafluoropropoxy)benzene | 5% |
| 2-fluoro-4-(2-(4-(4-propylcyclohexyl)phenyl)ethynyl)-1-(1,1,2,3,3,3-hexafluoropropoxy)benzene | 5% |
| 4'-(4-propylcyclohexyl)-3,5-difluoro-4-(1,1,2,3,3,3-hexafluoropropoxy)biphenyl) | 5% |
| 1-cyano-4-(4-(3-butenyl)cyclohexyl)benzene | 7% |
| 1-cyano-4-(4-(3-pentenyl)cyclohexyl)benzene | 7% |
| 1-cyano-4-(4-ethylcyclohexyl)benzene | 5% |
| 2-(4-ethylphenyl)-5-ethylpyrimidine | 6% |
| 2-(4-ethylphenyl)-5-propylpyrimidine | 6% |
| 2-(4-ethylphenyl)-5-butylpyrimidine | 6% |
| 1-methoxy-4-(2-(4-ethylphenyl)ethynyl)benzene | 3% |
| 1-methoxy-4-(2-(4-propylphenyl)ethynyl)benzene | 3% |
| 1-ethoxy-4-(2-(4-butylphenyl)ethynyl)benzene | 3% |
| 1-methoxy-4-(2-(4-pentylphenyl)ethynyl)benzene | 3% |
| 1-ethyl-4-(2-(2-fluoro-4-(4-propylcyclohexyl)phenyl)ethynyl)benzene | 5% |
| 1-propyl-4-(2-(2-fluoro-4-(4-propylcyclohexyl)phenyl)ethynyl)benzene | 5% |
| 2-(4-(4-ethylcyclohexyl)phenyl)-5-propylpyrimidine | 5% |
| 2-(4-(4-propylcydohexyl)phenyl)-5-ethylpyrimidine | 5% |
| 2-(4-(4-propylcyclohexyl)phenyl)-5-propylpyrimidine | 5% |
| 2-(4-(4-propylcydohexyl)phenyl)-5-butylpyrimidine | 5% |
| 1-propyl-4-(4-butylcyclohexyl)cyclohexane | 6% |

### Composition example 12

| | |
|---|---|
| 4-(2-(4-(4-ethylcyclohexyl)phenyl)ethynyl)-1-(1,1,2,3,3,3-hexafluoropropoxy)benzene (compound No. 267) | 5% |
| 4-(2-(4-(4-propylcyclohexyl)phenyl)ethynyl)-1-(1,1,2,3,3,3-hexafluoropropoxy)benzene (compound No. 283) | 5% |
| 4'-(4-propylcyclohexyl)-3-fluoro-4-(1,1,2,3,3,3-hexafluoropropoxy)biphenyl(compound No. 22) | 5% |
| 4-cyanophenyl 4-ethylbenzoate | 10% |
| 4-cyanophenyl 4-propylbenzoate | 4% |
| 2-(3,4-difluorophenyl)-5-propylpyrimidine | 10% |
| 2-(3,4-difluorophenyl)-5-pentylpyrimidine | 10% |
| 1-cyano-2-fluoro-4-(4-propylcyclohexyl)benzene | 4% |
| 1-cyano-2-fluoro-4-(4-(4-ethylcyclohexyl)cyclohexyl)benzene | 11% |
| 1-cyano-2-fluoro-4-(4-(4-propylcyclohexyl)cydohexyl)benzene | 11% |
| 2-(4'-fluorobiphenylyl)-5-propylpyrimidine | 5% |
| 2-(4'-fluorobiphenylyl)-5-butylpyrimidine | 5% |
| 4-fluorophenyl 4-(4-propylcyclohexyl)cyclohexanecarboxylate | 5% |
| 4-fluorophenyl 4-(4-pentylcyclohexyl)cyclohexanecarboxylate | 5% |

### Composition example 13

| | |
|---|---|
| 4'-(2-(4-propylcyclohexyl)ethyl)-4-(1,1,2,3,3,3-hexafluoropropoxy)biphenyl(compound No. 191) | 5% |
| 2'-fluoro-4'-(4-propylcyclohexyl)-4-(1,1,2,3,3,3-hexafluoropropoxy)biphenyl(compound No. 34) | 5% |
| 2'-fluoro-4'-(4-pentylcyclohexyl)-4-(1,1,2,3,3,3-hexafluoropropoxy)biphenyl(compound No. 36) | 5% |
| 1-cyano-4-(4-(3-butenyl)cyclohexyl)benzene | 7% |
| 1-cyano-4-(4-(3-pentenyl)cyclohexyl)benzene | 7% |
| 1-cyano-4-(4-ethylcyclohexyl)benzene | 7% |
| 1-cyano-4-(4-propylcyclohexyl)benzene | 7% |
| 1-methyl-4-(2-(4-ethylphenyl)ethynyl)benzene | 3% |
| 1-methyl-4-(2-(4-hexylphenyl)ethynyl)benzene | 6% |
| 1-butyl-4-(2-(4-butylphenyl)ethynyl)benzene | 3% |
| 1-methyl-4-(4-(4-propylcyclohexyl)cyclohexyl)benzene | 8% |
| 1-propyl-4-(4-(4-propylcyclohexyl)cyclohexyl)benzene | 5% |
| 1-ethyl-4-(2-(2-fluoro-4-(4-propylcyclohexyl)phenyl)ethynyl)benzene | 5% |
| 1-propyl-4-(2-(2-fluoro-4-(4-propylcyclohexyl)phenyl)ethynyl)benzene | 5% |
| 1-butyl-4-(2-(2-fluoro-4-(4-propylcyclohexyl)phenyl)ethynyl)benzene | 5% |
| 1-fluoro-4'-(4-ethylcyclohexyl)biphenyl | 6% |
| 1-fluoro-4'-(4-propylcyclohexyl)biphenyl | 6% |
| 1-fluoro-4'-(4-pentylcyclohexyl)biphenyl | 5% |

### Composition exmaple 14

| | |
|---|---|
| 4'-(2-(4-propylcyclohexyl)ethyl)-4-(1,1,2,3,3,3-hexafluoropropoxy)biphenyl(compound No. 191) | 5% |
| 2',6'-difluoro-4'-(4-propylcyclohexyl)-4-(1,1,2,3,3,3-hexafluoropropoxy)biphenyl(compoundNo. 47) | 5% |
| 2',6'-difluoro-4'-(4-pentylcyclohexyl)-4-(1,1,2,3,3,3-hexafluoropropoxy)biphenyl(compound No. 49) | 5% |
| 1-cyano-2-fluoro-4-(4-ethylcyclohexyl)benzene | 14% |
| 1-cyano-2-fluoro-4-(4-propylcyclohexyl)benzene | 12% |
| 2-(4-cyanophenyl)-5-butyl-1,3-dioxane | 10% |
| 1-cyano-4-(4-methoxymethylcyclohexyl)benzene | 10% |
| 4-butoxyphenyl 4-propylcyclohexanecarboxylate | 5% |
| 4-ethoxyphenyl 4-butylcyclohexanecarboxylate | 5% |
| 4-methoxyohenyl 4-pentylcyclohexanecarboxylate | 5% |
| 1,2-difluoro-4-(4-propylcyclohexyl)cyclohexyl)benzene | 10% |
| 1-fluoro-4-(4-(4-propylcyclohexyl)benzoyloxy)benzene | 8% |
| 1-fluoro-4-(4-(4-propylcyclohexyl)carbonyloxy)benzoyloxy)benzene | 6% |

### Composition exmaple 15

| | |
|---|---|
| 4'-(4-propylcyclohexyl)-3-fluoro-4-(1,1,2,3,3,3-hexafluoropropoxy)biphenyl | 5% |
| 4'-(4-pentylcyclohexyl)-3-fluoro-4-(1,1,2,3,3,3-hexafluoropropoxy)biphenyl | 5% |
| 4'-(2-(4-propylcyclohexyl)ethyl)-4-(1,1,2,3,3,3-hexafluoropropoxy)biphenyl(compound No. 191) | 5% |
| 1-methoxy-4-(4-propylcyclohexyl)cyclohexane | 7% |
| 1-fluoro-4-(4-pentylcyclohexyl)benzene | 7% |
| 1-propoxy-4-(4-propylcyclohexyl)cyclohexane | 5% |
| 1-methoxy-4-(4-pentylcyclohexyl)cydohexane | 4% |
| 1-trifluoromethoxy-4-(4-(4-ethylcyclohexyl)cyclohexyl)benzene | 4% |
| 1-difluoromethoxy-4-(4-(4-propylcyclohexyl)cyclohexyl)benzene | 3% |
| 1-trifluoromethoxy-4-(4-(4-propylcyclohexyl)cyclohexyl)benzene | 5% |
| 1-trifluoromethoxy-4-(4-(4-butylcyclohexyl)cyclohexyl)benzene | 4% |
| 1-difluoromethoxy-2,6-difluoro-4-(4-(4-propylcyclohexyl)cyclohexyl)benzene | 7% |
| 1-difluorolethoxy-4-(4-(4-pentylcyclohexyl)cyclohexyl)benzene | 4% |
| 1,2-difluoro-4-(2-(4-(4-propylcyclohexyl)cyclohexylethyl)benzene | 7% |
| 1-trifluoromethoxy-4-(4-(4-pentylcyclohexyl)cyclohexyl)benzene | 6% |
| 3,4-difluorophenyl 4-(4-propylcyclohexyl)cyclohexanecarboxylate | 4% |
| 1-difluoromethoxy-2,6-difluoro-4-(4-(4-pentylcyclohexyl)cyclohexyl)benzene | 10% |
| 1,2-difluoro-4-(2-(4-(4-pentylcyclohexyl)cyclohexylethyl)benzene | 8% |

### Composition example 16

| | |
|---|---|
| 4'-(1,1,2,3,3,3-hexafluoropropoxy)-4-(4-propylcyclohexyl)biphenyl(compound No. 1) | 5% |
| 4'-(1,1,2,3,3,3-hexafluoropropoxy)-4-(4-pentylcyclohexyl)biphenyl(compound No. 17) | 5% |
| 2-fluoro-4-(4-(4-propylcyclohexyl)cyclohexyl)-1-(1,1,2,3,3,3-hexafluoropropoxy)benzene | 5% |
| 2-fluoro-4-(4-(4-pentylcyclohexyl)cyclohexyl)-1-(1,1,2,3,3,3-hexafluoropropoxy)benzene | 5% |
| 1-fluoro-4-(4-pentylcyclohexyl)benzene | 5% |
| 1-fluoro-4-(4-hexylcyclohexyl)benzene | 5% |
| 1-fluoro-4-(4-heplylcyclohexyl)benzene | 5% |
| 1-trifluoromethoxy-4-(4-(4-ethylcyclohexyl)cyclohexyl)benzene | 5% |
| 1-trifluoromethoxy-4-(4-(4-propylcyclohexyl)cyclohexyl)benzene | 7% |
| 1,2-difluoro-4'-(4-propylcyclohexyl)biphenyl | 9% |
| 1-trifluoromethoxy-4-(4-(4-butylcyclohexyl)cyclohexyl)benzene | 4% |
| 1-trifluoromethoxy-4-(2-(4-(4-propylcyclohexyl)cyclohexyl)ethyl)benzene | 6% |
| 1-trifluoromethoxy-4(4-(4-pentylcyclohexyl)cyclohexylbenzene | 8% |
| 1,2-difluoro-4'-(4-pentylcyclohexyl)biphenyl | 12% |
| 1-ethyl-2-fluoro-4'-(4-pentylcyclohexyl)biphenyl | 8% |
| 2-fluoro-4-(4-propylcyclohexyl)-4'-(4-propylcyclohexyl)biphenyl | 2% |
| 2-fluoro-4-(4-pentylcyclohexyl)-4'-(4-propylcyclohexyl)biphenyl | 2% |
| 2-fluoro-4-(4-pentylcyclohexyl)-4'-(4-pentylcyclohexyl)biphenyl | 2% |

### Composition example 17

| | |
|---|---|
| 2',6'-difluoro-4'-(4-propylcyclohexyl)-4-(1,1,2,3,3,3-hexafluoropropoxy)biphenyl(compound No. 47) | 5% |
| 2',6'-difluoro-4'-(4-pentylcyclohexyl)-4-(1,1,2,3,3,3-hexafluoropropoxy)biphenyl(compound No. 49) | 5% |
| 1-cyano-4-(4-(2-propenyl)cyclohexyl)benzene | 3% |
| 4'-butyl-4-ethylbiphenyl | 3% |
| 4'-propyl-4-cyanobiphenyl | 3% |
| 1-cyano-2-fluoro-4-(4-ethylcyclohexyl)benzene | 3% |
| 2-(3,4-difluorophenyl)-5-propylpyrimidine | 3% |
| 1-ethoxy-4-(2-(4-propylcyclohexyl)ethyl)benzene | 5% |
| 4'-pentyl-4-cyanobiphenyl | 8% |
| 4-cyanophenyl 4-propylbenzoate | 3% |
| 4-ethylphenyl 4-butylcyclohexanecarboxylate | 11% |
| 1-ethoxy-4-(2-(4-pentylycyclohexyl)ethyl)benzene | 12% |
| 4-methoxyphenyl 4-pentylcyclohexanecarboxylate | 16% |
| 4-propoxyphenyl 4-pentylcyclohexanecarboxylate | 10% |
| 2-(4-cyanophenyl)-5-(4-butylphenyl)pyrimidine | 3% |
| 1"-pentyl-4-cyanoterphenyl | 5% |
| 1-butyl-4-(2-(4-(4-pentylcycmphexyl)phenyl)elhyl)benzene | 6% |
| 2-(4-pentylphenyl)-5-(4-butylphenyl)pyrimidine | 3% |
| 1-propyl-4-(2-(4-(4-(4-pentylcyclohexyl)phenyl)phenyl)ethyl)cyclohexane | 3% |

### Composition example 18 (for comparison purposes)

| | |
|---|---|
| 4'-(4-propylcyclohexyl)-3-fluoro-4-(1,1,2,3,3,3-hexafluoropropoxy)biphenyl | 5% |
| 4'-(4-pentylcyclohexyl)-3-fluoro-4-(1,1,2,3,3,3-hexafluoropropoxy)biphenyl | 5% |
| 1,2-difluorophenyl 4-butylcyclohexanecarboxylate | 11% |
| 1,2-difluorophenyl 4-pentylcyclohexanecarboxylate | 5% |
| 1-cyano-2-fluorophenyl 4-ethylbenzoate | 4% |
| 1-cyano-2-fluoro-4-(4-(3-methoxypropyl)cyclohexyl)benzene | 11% |
| 1-cyano-2-fluorophenyl 4-propylbenzoate | 4% |
| 1-cyano-2-fluorophenyl 4-butylbenzoate | 5% |
| 1-cyano-2-fluorophenyl 4-pentylbenzoate | 5% |
| 1,2-difluorophenyl 4-(4-propylcyclohexyl)cyclohexanecarboxylate | 4% |
| 1,2-difluorophenyl 4-(4-pentylcyclohexyl)cyclohexanecarboxylate | 5% |
| 1-cyano-2-fluorophenyl 4-(4-ethylcyclohexyl)benzoate | 5% |
| 1-ethyl-4-(2-(4-(4-propylcyclohexyl)phenyl)ethynyl)benzene | 14% |
| 1-cyano-2-fluorophenyl 4-(4-propylcyclohexyl)benzoate | 9% |
| 1-cyano-2-fluorophenyl 4-(4-butylcyclohexyl)benzoate | 4% |
| 1-cyano-2-fluorophenyl 4-(4-pentylcyclohexyl)benzoate | 4% |

### Composition example 19

| | |
|---|---|
| 2-fluoro-4-(2-(4-(4-ethylcyclohexyl)phenyl)ethynyl)-1-(1,1,2,3,3,3-hexafluoropropoxy)benzene | 5% |
| 4-(2-(4-(4-ethylcyclohexyl)phenyl)ethynyl)-1-(1,1,2,3,3,3-hexafluoropropoxy)benzene (compound No. 267) | 5% |
| 2',6'-difluoro-4-(4-(4-propylcyclohexyl)cyclohexyl)-1-(1,1,2,3,3,3-hexafluoropropoxy)benzene | 5% |
| 2',6'-difluoro-4-(4-(4-pentylcyclohexyl)cyclohexyl)-1-(1,1,2,3,3,3-hexafluoropropoxy)benzene | 5% |
| 1-ethyl-4-(4-propylcyclohexyl)benzene | 6% |
| 1-cyano-4-(4-ethylcyclohexyl)benzene | 10% |
| 1-cyano-4-(4-propylcyclohexyl)benzene | 10% |
| 1-cyano-4-(4-butylcyclohexyl)benzene | 10% |
| 1-cyano-4-(4-pentylcyclohexyl)benzene | 10% |
| 1-cyano-4-(4-heptylcyclohexyl)benzene | 10% |
| 1-ethyl-4'-(4-propylcyclohexyl)biphenyl | 12% |
| 1-ethyl-4'-(4-pentylcyclohexyl)biphenyl | 4% |
| 1-propyl-4'-(4-propylcyclohexyl)biphenyl | 3% |
| 4-(4-propylcyclohexyl)-4'-(4-pentylcyclohexyl)biphenyl | 5% |

### Composition example 20

A composition consisting of the same components as those in Composition example 1, except that 4'-(1,1,2,3,3,3-hexafluoropropoxy)-4-(4-propylcyclohexyl)biphenyl (compound No. 1) was replaced by 4'-(4-methoxymethylcyclohexyl)-3-fluoro-4-(1,1,2,3,3,3-hexafluoropropyloxy)biphenyl.

### Composition example 21

A composition consisting of the same components as those in composition example 8, except that 4-(2-(4-(4-ethylcyclohexyl)phenyl)ethynyl)-1-(1,1,2,3,3,3-hexafluoropropyloxy)benzene (compound No. 267) was replaced by 4-(2-(4-(4-methoxymethylcyclohexyl)cyclohexyl)ethynyl-1-(1,1,2,3,3,3-hexafluoropropyloxy)benzene (compound No. 456).

### Composition example 22

A composition consisting of the same components as those in composition example 9, except that 4-(2-(4-(4-propylcyclohexyl)phenyl)ethynyl)-1-(1,1,2,3,3,3-hexafluoropropoxy)benzene (compound No. 283) was replaced by4-(2-(4-(4-methoxymethoxycyclohexyl)cyclohexyl)ethynyl)-1-(1,1,2,3,3,3-hexafluoropropyloxy)benzene (compound No. 457).

### Composition example 23

A composition consisting of the same components as those in composition example 15, except that 4'-(2-(4-propylcyclohexyl)ethyl)-4-(1,1,2,3,3,3-hexafluoropropoxy)biphenyl(compound No. 191) was replaced by 2',6-difluoro-4'-(2-(4-methoxymethylcyclohexyl)ethyl-3,5-difluoro-4-(1,1,2,3,3,3-hexafluoropropyl)biphenyl (compound No. 445).

### (Example)

The present invention will be described in more detail by way of Examples. Hereinafter, 1,4-cyclohexylene and 1,2-ethenylene in side chains or central linkages all represent transconformation. Further, the unit of the phase transition points are all represented by °C.

### Example 1

Preparation of 4'-(1,1,2,3,3,3-hexafluoropropoxy)-4-(4-propylcyclohexyl)biphenyl (compound No. 1) (a compound of the formula (1) wherein R₁ = propyl group; X = covalent bond; Y₁, Y₂ =hydrogen atom; ℓ = m = 1; n = 0; p = 1; ring A =cyclohexane ring; and ring B = benzene ring)

A tetrahydrofuran (hereinafter abbreviated to THF) (60 mℓ) solution of 4-(4-propylcyclohexyl)bromobenzene (5.6 g, 20 mmol) prepared according to a process of Japanese patent application laid-open No. Sho 56-103120 was added to a THF (5 mℓ) solution of sufficiently dried Mg (0.54 g, 22 mmol), followed by heating the mixture under reflux for 3 hours, adding a THF solution (0.5 mol solution) of zinc chloride (44 mℓ, 22 mmol) on an ice water bath, stirring at room temperature for 30 minutes, further adding commercially available 4-(1,1,2,3,3,3-hexafluoropropoxy)bromobenzene (6.46 g, 20 mmol) and tetrakis(triphenylphosphine) palladium (0) (680 mg), heating the mixture under reflux for 7.5 hours allowing the resulting material to cool, adding to 3N hydrochloric acid (100 mℓ), extracting with heptane (400 mℓ), washing the organic layer with water, washing with a saturated aqueous solution of sodium hydrogen carbonate, three times washing with water, drying over anhydrous magnesium sulfate, distilling off the solvent under reduced pressure, distilling the residue under reduced pressure, subjecting the thus obtained white solids to column chromatography (using a mixed solvent of hexane/toluene (10/1) as an eluent), and recrystallizing from a mixed solvent of methanol/ethanol (9 mℓ), to obtain the captioned compound (2.18 g) (yield: 24%).
Boiling point: 195 - 205°C (2 mmHg, 270 N/m²)
Cr 118.2°C SB 176.4°C SA 181.9°C Iₛₒ

The following compounds are prepared in the same manner as above:
Compound No.
2. 4'-propyl-4-(1,1,2,3,3,3-hexafluoropropoxy)biphenyl
3. 4'-butyl-4-(1,1,2,3,3,3-hexafluoropropoxy)biphenyl
4. 4'-pentyl-4-(1,1,2,3,3,3-hexafluoropropoxy)biphenyl
5. 2'-fluoro-4'-propyl-4-(1,1,2,3,3,3-hexafluoropropoxy)biphenyl
6. 2'fluoro-4'-pentyl-4-(1,1,2,3,3,3-hexafluoropropoxy)biphenyl
7. 4'-propyl-3-fluoro-4-(1,1,2,3,3,3-hexafluoropropoxy)biphenyl
8. 4'-pentyl-3-fluoro-4-(1,1,2,3,3,3-hexafluoropropoxy)biphenyl
9. 4'-propyl-3-chloro-4-(1,1,2,3,3,3-hexafluoropropoxy)biphenyl
10. 4'-butyl-3-chloro-4-(1,1,2,3,3,3-hexafluoropropoxy)biphenyl
11. 4'-pentyl-3-methyl-4-(1,1,2,3,3,3-hexafluoropropoxy)biphenyl

14. 4'-(4-methylcyclohexyl)-4-(1,1,2,3,3,3-hexafluoropropoxy)biphenyl
15. 4'-(4-ethylcyclohexyl)-4-(1,1,2,3,3,3-hexafluoropropoxy)biphenyl
16. 4'-(4-butylcyclohexyl)-4-(1,1,2,3,3,3-hexafluoropropoxy)biphenyl
17. 4'-(4-pentylcyclohexyl)-4-(1,1,2,3,3,3-hexafluoropropoxy)biphenyl Cr 176.5°C SB 181.5°C Iₛₒ
18. 4'-(4-pentylcyclohexyl)-4-(1,1,2,3,3,3-hexafluoropropyl)biphenyl
19. 4'-(4-hexylcyclohexyl)-4-(1,1,2,3,3,3-hexafluoropropoxy)biphenyl
20. 4'-(4-methylcyclohexyl)-3-chloro-4-(1,1,2,3,3,3-hexafluoropropoxy)biphenyl
21. 4'-(4-ethylcyclohexyl)-3-methyl-4-(1,1,2,3,3,3-hexafluoropropoxy)biphenyl
23. 4'-(4-propylcyclohexyl)-3-fluoro-4-(1,1,2,3,3,3-hexafluoropropyl)biphenyl
26. 4'-(4-pentylcyclohexyl)-3-fluoro-4-(1,1,2,3,3,3-hexafluoropropyl)biphenyl
32. 2'-fluoro-4'-(4-methylcyclohexyl)-4-(1,1,2,3,3,3-hexafluoropropoxy)biphenyl
33. 2'-fluoro-4'-(4-ethylcyclohexyl)-4-(1,1,2,3,3,3-hexafluoropropoxy)biphenyl
34. 2'-fluora-4'-(4-propylcyclohexyl)-4-(1,1,2,3,3,3-hexafluoropropoxy)biphenyl Cr 91.7 (SB 90.8) SA 130.8 N 37.0 Iₛₒ
35. 2'-fluoro-4'-(4-butylcyclohexyl)-4-(1,1,2,3,3,3-hexafluoropropoxy)biphenyl
36. 2'-fluoro-4'-(4-pentylcyclohexyl)-4-(1,1,2,3,3,3-hexafluoropropoxy)biphenyl
37. 2'-fluoro-4'-(4-pentylcyclohexyl)-4-(1,1,2,3,3,3-hexafluoropropyl)biphenyl
38. 2'-fluoro-4'-(4-hexylcyclohexyl)-4-(1,1,2,3,3,3-hexafluoropropoxy)biphenyl
40. 2'-fluoro-4'-(4-ethylcyclohexyl)-3-chloro-4-(1,1,2,3,3,3-hexafluoropropoxy)biphenyl
42. 2'-fluoro-4'-(4-butylcyclohexyl)-3-methyl-4-(1,1,2,3,3,3-hexafluoropropoxy)biphenyl
45. 2',6'-difluoro-4'-(4-methylcyclohexyl)-4-(1,1,2,3,3,3-hexafluoropropoxy)biphenyl
46. 2',6'-difluoro-4'-(4-ethylcyclohexyl)-4-(1,1,2,3,3,3-hexafluoropropoxy)biphenyl
47. 2',6'-difluoro-4'-(4-propylcyclohexyl)-4-(1,1,2,3,3,3-hexafluoropropoxy)biphenyl
48. 2',6'-difluoro-4'-(4-butylcyclohexyl)-4-(1,1,2,3,3,3-hexafluoropropoxy)biphenyl
49. 2',6'-difluoro-4'-(4-pentylcyclohexyl)-4-(1,1,2,3,3,3-hexafluoropropoxy)biphenyl Cr 94.3 SA 95.6 N 108.0 Iₛₒ
50. 2',6'-difluoro-4'-(4-pentylcyclohexyl)-4-(1,1,2,3,3,3-hexafluoropropyl)biphenyl
51. 2',6'-difluoro-4'-(4-hexylcyclohexyl)-4-(1,1,2,3,3,3-hexafluoropropoxy)biphenyl
52. 2',6'-difluoro-4'-(4-methylcyclohexyl)-3-methyl-4-(1,1,2,3,3,3-hexafluoropropoxy)biphenyl
53. 2',6'-difluoro-4'-(4-ethylcyclohexyl)-3-chloro-4-(1,1,2,3,3,3-hexafluoropropoxy)biphenyl
55. 2'.6'-difluoro-4'-(4-butylcyclohexyl)-3-chloro-4-(1,1,2,3,3,3-hexafluoropropoxy)biphenyl
58. 4"-propyl-4-(1,1,2,3,3,3-hexafluoropropoxy)terphenyl
59. 4"-pentyl-4-(1,1,2,3,3,3-hexafluoropropoxy)terphenyl
62. 4"-propyl-2'-fluoro-4-(1,1,2,3,3,3-hexafluoropropoxy)terphenyl
66. 4"-propyl-2',6'-difluoro-4-(1,1,2,3,3,3-hexafluoropropoxy)terphenyl
68. 4"-propyl-4-(1,1,2,3,3,3-hexafluoropropyl)terphenyl
69. 4"-propyl-2',6'-difluoro-3,5-difluoro-4-(1,1,2,3,3,3-hexafluoropropyl)terphenyl
70. 4"-propyl-2',6'-difluoro-4-(1,1,2,3,3,3-hexafluoropropyl)terphenyl
71. 4'-(4-(4-methylcyclohexyl)cyclohexyl)-4-(1,1,2,3,3,3-hexafluoropropoxy)biphenyl
72. 2'-fluoro-4'-(4-(4-methylcyclohexyl)cyclohexyl)-4-(1,1,2,3,3,3-hexafluoropropoxy)biphenyl
73. 4'-(4-(4-propylcyclohexyl)cyclohexyl)-4-(1,1,2,3,3,3-hexafluoropropoxy)biphenyl
74. (2'-fluoro-4'-(4-(4-propylcyclohexyl)cyclohexyl-4-(1,1,2,3,3,3-hexafluoropropoxy)biphenyl

77. 4'-(4-(4-pentylcyclohexyl)cydohexyl)-4-(1,1,2,3,3,3-hexafluoropropoxy)biphenyl
78. 2'-fluoro-4'-(4-(4-pentylcyclohexyl)cyclohexyl)-4-(1,1,2,3,3,3-hexafluoropropoxy)biphenyl

81. 4'-(4-(4-methylcyclohexyl)cyclohexyl)-3-fluoro-4-(1,1,2,3,3,3-hexafluoropropyl)biphenyl
82. 2'-fluoro-4'-(4-(4-propylcyclohexyl)cyclohexyl)-3,5-difluoro-4-(1,1,2,3,3,3-hexafluoropropyl)biphenyl
83. 2',6'-difluoro-4'-(4-(4-propylcyclohexyl)cyclohexyl)-3,5-difluoro-4-(1,1,2,3,3,3-hexafluoropropyl)biphenyl
84. 4"-(4-propylcyclohexyl)-4-(1,1,2,3,3,3-hexafluoropropoxy)terphenyl
86. 4"-(4-propylcyclohexyl)-4-(1,1,2,3,3,3-hexafluoropropyl)terphenyl

### Example 2

### Preparation of 4-(4-(4-propylcyclohexyl)cyclohexyl)-1-(1,1,2,3,3,3-hexafluoropropoxy)benzene (compound No. 87)

### (a compound of the formula (1) wherein R₁ = propyl group; X = covalent bond; Y₁ and Y₂: hydrogen atom; ℓ = m = 1, n = 0, p = 1; and rings A and B: cyclohexane ring)

A mixture of 4-(4-propylcyclohexyl)bromocyclohexane (5.6 g, 20 mmol), prepared by reacting 4-(4-propylcyclohexyl ) cyclohexanol obtained according to methods disclosed in Japanese patent application laid-open Nos. Sho 57-64626, and Sho 57-165328, using triphenylphosphine dibromide, according to a method disclosed in Journal of the American Chemical Society, 86, 964 (1964), zinc bromide (2.23 g, 10 mmol), lithium (280 mg), toluene (40 mℓ) and THF (3 mℓ), was irradiated by ultrasonic wave at 0°C for one hour, followed by adding to the resulting black solution, 4-(1,1,2,3,3,3-hexafluoropropoxy)bromobenzene (6.46 g, 20 mmol) and bis(triphenylphosphine)palladium (II) chloride (600 mg), heating the mixture under reflux for 5 hours, allowing the resulting material to cool, feeding it into 3N-HCℓ. (100 mℓ), extracting with heptane, washing the organic layer with water, washing with a saturated aqueous solution of sodium hydrogen carbonate, further three times washing with water, drying over anhydrous magnesium sulfate, distilling off the solvent under reduced pressure, distilling the residue under reduced pressure, subjecting the distillate to column chromatography and recrystallizing to obtain the captioned compound. Boiling point: 232 - 236°C (1 mmHg, 130 N/m²).

The following compounds are prepared in the same manner as above:
Compound No.
88. 4-(4-methylcyclohexyl)-1-(1,1,2,3,3,3-hexafluoropropyl)benzene
89. 4-(4-methylcyclohexyl)-1-(1,1,2,3,3,3-hexafluoropropoxy)benzene
90. 4-(4-ethylcyclohexyl)-1-(1,1,2,3,3,3-hexafluoropropoxy)benzene
91. 4-(4-propylcyclohexyl)-1-(1,1,2,3,3,3-hexafluoropropoxy)benzene
92. 4-(4-propylcyclohexyl)-1-(1,1,2,3,3,3-hexafluoropropyl)benzene
93. 4-(4-pentylcyclohexyl)-1-(1,1,2,3,3,3-hexafluoropropyl)benzene
94. 4-(4-hexylcyclohexyl)-1-(1,1,2,3,3,3-hexafluoropropyl)benzene
95. 4-(4-(3-propenyl)cyclohexyl)-1-(1,1,2,3,3,3-hexafluoropropyl)benzene
96. 4-(4-(3-butenyl)cyclohexyl)-1-(1,1,2,3,3,3-hexafluoropropyl)benzene
97. 4-(4-butylcyclohexyl)-1-(1,1,2,3,3,3-hexafluoropropoxy)benzene
98. 4-(4-pentylcyclohexyl)-1-(1,1,2,3,3,3-hexafluoropropoxy)benzene
99. 4-(4-pentylcyclohexyl)-1-(1,1,2,3,3,3-hexafluoropropyl)benzene
100. 4-(4-hexylcyclohexyl)-1-(1,1,2,3,3,3-hexafluoropropoxy)benzene
101. 4-(4-heptylcyclohexyl)-1-(1,1,2,3,3,3-hexafluoropropoxy)benzene Oily substance
105. 2-chloro-4-(4-butylcyclohexyl)-1-(1,1,2,3,3,3-hexafluoropropoxy)benzene

108. 2-chloro-4-(4-heptylcyclohexyl)-1-(1,1,2,3,3,3-hexafluoropropoxy)benzene
109. 4-(4-(4-methylcyclohexyl)cyclohexyl)-1-(1,1,2,3,3,3-hexafluoropropoxy)benzene
110. 4-(4-(4-propylcyclohexyl)cyclohexy))-1-(1,1,2,3,3,3-hexafluoropropoxy)benzene
111. 4-(4-(4-pentylcyclohexyl)cyclohexyl)-1-(1,1,2,3,3,3-hexafluoropropoxy)benzene
112. 4-(4-(4-octylcyclohexyl)cyclohexyl)-1-(1,1,2,3,3,3-hexafluoropropoxy)benzene
113. 4-(4-(4-hexylcyclohexyl)cyclohexyl)-1-(1,1,2,3,3,3-hexafluoropropoxy)benzene

116. 2-chloro-4-(4-(4-propylcyclohexyl)cyclohexyl)-1-(1,1,2,3,3,3-hexafluoropropoxy)benzene
117. 2-chloro-4-(4-(4-pentylcyclohexyl)cyclohexyl)-1-(1,1,2,3,3,3-hexafluoropropoxy)benzene

120. 4-(4-(4-(4-propylcyclonexyl)cyclonexyl)cyclohexyl)-1-(1,1,2,3,3,3-hexafluoropropoxy)benzene
121. 4-(4-(4-(4-pentylcyclohexyl)cyclohexyl)cyclohexyl)-1-(1,1,2,3,3,3-hexafluoropropoxy)benzene
122. 4-(4-(4-(4-heptylcyclohexyl)cyclohexyl)cyclohexyl)-1-(1,1,2,3,3,3-hexafluoropropoxy)benzene
123. 4-(4-(4-(4-nonylcyclohexyl)cyclohexyl)cyclohexyl)-1-(1,1,2,3,3,3-hexafluoropropoxy)benzene
127. 2-chloro-4-(4-(4-(4-pentylcyclohexyl)cyclohexyl)cyclohexyl)-1-(1,1,2,3,3,3-hexafluoropropoxy)benzene

### Example 3

### Preparation of 4-(2-(4-(4-propylcyclohexyl)cyclohexyl)ethyl-1-(1,1,2,3,3,3-hexafluoropropoxy)benzene (compound No. 128)

### (a compound of the formula (1) wherein R₁ = propyl group; X = -CH₂CH₂-; Y₁ and Y₂ = hydrogen atom; ℓ = m = 1, n = 0, p = 1; and rings A and B = cyclohexane ring)

A mixture of 2-(4-(4-propylcyclohexyl)cyclohexyl)ethylbromide (6.86 g, 20 mmol) obtained by conventionally increasing the carbon number of 4-(4-propylcyclohexyl)cyclohexane carboxylic acid prepared according to a method disclosed in Angewandte Chemie 89, 103 (1978), zinc bromide (2.23 g, 10 mmol), lithium (280 mg), toluene (50 mℓ) and THF (4 mℓ) was irradiated by ultrasonic wave at 0°C for one hour, followed by adding to the resulting black solution, 4-(1,1,2,3,3,3-hexafluoropropoxy)bromobenzene (6.46 g, 20 mmol) and bis(triphenylphosphine)palladium (II) chloride (600 mg), heating the mixture under reflux for 5 hours, allowing the resulting material to cool, feeding it into 3N-HCℓ (100 mℓ), extracting with heptane, washing the organic layer with water, washing with a saturated sodium hydrogen carbonate, further three times washing with water, drying over anhydrous magnesium sulfate, distilling off the solvent under reduced pressure, distilling the residue under reduced pressure, subjecting to column chromatography and recrystallizing, to obtain the captioned compound.
Boiling Point: 238 - 245°C (1 mmHg, 130 N/m²)

The following compounds are prepared in the same manner as above:
Compound No.
129. 4-(2-(4-ethylphenyl)ethyl)-1-(1,1,2,3,3,3-hexafluoropropoxy)benzene
130. 4-(2-(4-propylphenyl)ethyl)-1-(1,1,2,3,3,3-hexafluoropropoxy)benzene
131. 4-(2-(4-pentylphenyl)ethyl)-1-(1,1,2,3,3,3-hexafluoropropoxy)benzene

133. 2-chloro-4-(2-(4-ethylphenyl)ethyl)-1-(1,1,2,3,3,3-hexafluoropropoxy)benzene
134. 4-(2-(4-ethylcyclohexyl)ethyl)-1-(1,1,2,3,3,3-hexafluoropropoxy)benzene
135. 4-(2-(4-propylcyclohexyl)ethyl)-1-(1,1,2,3,3,3-hexafluoropropoxy)benzene
136. 4-(2-(4-pentylcyclohexyl)ethyl)-1-(1,1,2,3,3,3-hexafluoropropoxy)benzene

138. 2-chloro-4-(2-(4-ethylcyclohexyl)ethyl)-1-(1,1,2,3,3,3-hexafluoropropoxy)benzene
139. 4-(2-(4-(4-ethylcyclohexyl)cyclohexyl)ethyl)-1-(1,1,2,3,3,3-hexafluoropropoxy)benzene
140. 4-(2-(4-(4-butylcyclohexyl)cyclohexyl)ethyl)-1-(1,1,2,3,3,3-hexafluoropropoxy)benzene
141. 4-(2-(4-(4-pentylcyclohexyl)cyclohexyl)ethyl)-1-(1,1,2,3,3,3-hexafluoropropoxy)benzene
142. 4-(2-(4-(4-pentylcyclohexyl)cyclohexyl)ethyl)-1-(1,1,2,3,3,3-hexafluoropropyl)benzene
143. 2-chloro-4-(2-(4-(4-ethylcyclohexyl)cyclohexyl)ethyl)-1-(1,1,2,3,3,3-hexafluoropropoxy)benzene

147. 4-(2-(4-(4-ethylcyclohexyl)phenyl)ethyl)-1-(1,1,2,3,3,3-hexafluoropropoxy)benzene
148. 4-(2-(4-(4-propylcyclohexyl)phenyl)ethyl)-1-(1,1,2,3,3,3-hexafluoropropoxy)benzene
149. 4-(2-(4-(4-butylcyclohexyl)phenyl)ethyl)-1-(1,1,2,3,3,3-hexafluoropropoxy)benzene
150. 4-(2-(4-(4-pentylcyclohexyl)phenyl)ethyl)-1-(1,1,2,3,3,3-hexafluoropropoxy)benzene
151. 2-chloro-4-(2-(4-(4-ethylcyclohexyl)pehnyl)ethyl)-1-(1,1,2,3,3,3-hexafluoropropoxy)benzene

154. 4-(2-(2-fluoro-4-(4-ethylcyclohexyl)phenyl)ethyl)-1-(1,1,2,3,3,3-hexafluoropropyloxy)benzene
155. 4-(2-(2-fluoro-4-(4-propylcyclohexyl)phenyl)ethyl)-1-(1,1,2,3,3,3-hexafluoropropoxy)benzene
156. 4-(2-(2-fluoro-4-(4-butylcyclohexyl)phenyl)ethyl)-1-(1,1,2,3,3,3-hexafluoropropoxy)benzene
157. 4-(2-(2-fluoro-4-(4-pentylcyclohexyl)phenyl)ethyl)-1-(1,1,2,3,3,3-hexafluoropropoxy)benzene
158. 2-chloro-4-(2-(2-fluoro-4-(4-ethylcyclohexyl)phenyl)ethyl)-1-(1,1,2,3,3,3-hexafluoropropoxy)benzene

160. 4-(2-(4-(4-propylphenyl)phenyl)ethyl)-1-(1,1,2,3,3,3-hexafluoropropoxy)benzene
161. 4-(2-(4-(4-pentylphenyl)phenyl)ethyl)-1-(1,1,2,3,3,3-hexafluoropropoxy)benzene

163. 2-chloro-4-(2-(4-(4-propylphenyl)phenyl)ethyl)-1-(1,1,2,3,3,3-hexafluoropropoxy)benzene
164. 2-methyl-4-(2-(4-(4-propylphenyl)phenyl)ethyl)-1-(1,1,2,3,3,3-hexafluoropropoxy)benzene

166. 4-(2-(4-(4-propylphenyl)cyclohexyl)ethyl)-1-(1,1,2,3,3,3-hexafluoropropoxy)benzene
167. 4-(2-(4-(4-pentylphenyl)cyclohexyl)ethyl)-1-(1,1,2,3,3,3-hexafluoropropoxy)benzene
168. 2-methyl-4-(2-(4-(4-propylphenyl)cyclohexyl)ethyl)-1-(1,1,2,3,3,3-hexafluoropropoxy)benzene

172. 2-chloro-4-(2-(4-(2-fluoro-4-propylphenyl)cyclohexyl)ethyl)-1-(1,1,2,3,3,3-hexafluoropropoxy)benzene
173. 4'-(2-(4-propylphenyl)ethyl)-4-(1,1,2,3,3,3-hexafluoropropoxy)biphenyl

177. 4'-(2-(4-propylphenyl)ethyl)-4-(1,1,2,3,3,3-hexafluoropropyl)biphenyl
178. 4'-(2-(4-propylphenyl)ethyl-3,5-difluoro-4-(1,1,2,3,3,3-hexafluoropropyl)biphenyl
179. 4'-(2-(4-propylcyclohexyl)ethyl)-4-(1,1,2,3,3,3-hexafluoropropoxy)biphenyl

183. 4'-(2-(4-propylphenyl)ethyl)-3-fluoro-4-(1,1,2,3,3,3-hexafluoropropyl)biphenyl
184. 2'-fluoro-4'-(2-(4-propylphenyl)ethyl)-3,5-difluoro-4-(1,1,2,3,3,3-hexafluoropropyl)biphenyl
185. 4'-(2-(4-(4-propylcyclohexyl)phenyl)ethyl)-4-(1,1,2,3,3,3-hexafluoropropoxy)biphenyl

188. 4'-(2-(4'-propylbiphenylyl)ethyl)-4-(1,1,2,3,3,3-hexafluoropropoxy)biphenyl
189. 4'-(2-(4'-propylbiphenylyl)ethyl)-4-(1,1,2,3,3,3-hexafluoropropyl)biphenyl

### Example 4

### Preparation of 4'-(2-(4-propylcyclohexyl)ethyl-4-(1,1,2,3,3,3-hexafluoropropoxy)biphenyl (compound No. 191)

### (a compound of the formula (1), wherein R₁ = propyl group; X = -CH₂CH₂-; Y₁ and Y₂ = hydrogen atom; ℓ= 1, m=0, p = 1; ring A: cyclohexane ring; and ring C: benzene ring)

A hexane solution of n-butyllithium (12.5 mℓ, 20 mmol) (1.60 mol solution) was dropwise added to a THF (80 mℓ) solution of 4-(2-(4-propylcyclohexyl)ethyl)iodobenzene (5.94 g, 16.7 mmol) prepared according to the method disclosed in the Journal of Practical Chemistry, 320, 191 (1978), at -50°C or lower, followed by stirring at the same temperature for 2 hours, adding a THF solution (0.5 mol solution) of zinc chloride (40 mℓ, 20 mmol), stirring the mixture at room temperature for 45 minutes, further adding 4-(1,1,2,3,3,3-hexafluoropropoxy)bromobenzene (6.46 g, 20 mmol) and tetrakis(triphenylphosphine)palladium (0) (5,000 mg), heating the mixture under reflux for 2.5 hours, allowing the resulting material to cool, feeding it into 3-N HCℓ (400 mℓ), extracting with toluene (200 mℓ), washing the organic layer with water, washing with a saturated aqueous solution of sodium hydrogen carbonate, further three times washing with water, drying over anhydrous magnesium sulfate, distilling off the solvent under reduced pressure, subjecting the residue to column chromatography (eluent: hexane), and recrystallizing from a mixed solvent (10 mℓ) of methanol with ethanol, to obtain the captioned compound (2.39 g) (yield: 30%).
Cr 139.7°C (SB 139.5°C) SA 144.7°C N 146.5°C Iₛₒ

The following compounds are prepared in the same manner as above:
192. 4'-(2-(4-butylcyclohexyl)ethyl)-4-(1,1,2,3,3,3-hexafluoropropoxy)biphenyl
193. 4'-(2-(4-pentylcyclohexyl)ethyl)-4-(1,1,2,3,3,3-hexafluoropropoxy)biphenyl
194. 4'-(2-(4-hexylcyclohexyl)ethyl)-4-(1,1,2,3,3,3-hexafluoropropoxy)biphenyl
196. 4'-(2-(4-butylcyclohexyl)ethyl)-3-chloro-4-(1,1,2,3,3,3-hexafluoropropoxy)biphenyl
198. 4'-(2-(4-hexylcyclohexyl)ethyl)-3-methyl-4-(1,1,2,3,3,3-hexafluoropropoxy)biphenyl
199. 4-(2-(4-propylcyclohexyl)ethyl)cyclohexyl-1-(1,1,2,3,3,3-hexafluoropropoxy)benzene
200. 4-(2-(4-propylcyclohexyl)ethyl)cyclohexyl-1-(1,1,2,3,3,3-hexafluoropropyl)benzene
201. 4-(2-(4-pentylcyclohexyl)ethyl)cyclohexyl-1-(1,1,2,3,3,3-hexafluoropropoxy)benzene
202. 4-(2-(4-pentylcyclohexyl)ethyl)cyclohexyl-1-(1,1,2,3,3,3-hexafluoropropyl)benzene
206. 4'-(2-(4-(4-pentylcyclohexyl)cyclohexyl)ethyl)-4-(1,1,2,3,3,3-hexafluoropropoxy)biphenyl

### Example 5

### Preparation of 4-(2-(4-(4-propylcyclohexyl)cyclohexyl)ethenyl)-1-(1,1,2,3,3,3-hexafluoropropoxy)benzene (compound No. 211)

### (a compound of the formula (1) wherein R₁ = propyl group; X = -CH=CH-; Y₁ and Y₂ = hydrogen atom; ℓ = m = 1, n = 0, p = 1; and rings A and B = cyclohexane ring)

An ultrasonic wave was irradiated at 0°C for onr hour, to a mixture of 2-(4-(4-propylcyclohexyl)cyclohexyl)iodized ethenyl (6.82 g, 20 mmol) prepared according to the method disclosed in Journal of the American Chemical Society, 95, 5786 (1973), zinc bromide (2.23 g, 10 mmol), lithium (280 mg), toluene (50 mℓ) and THF (4 mℓ), followed by adding to the resulting black solution, 4-(1,1,2,3,3,3-hexafluoropropoxy)bromobenzene (6.46 g, 20 mmol) and bis(triphenylphosphine)palladium (II) chloride (600 mg), heating the mixture under reflux for 5 hours, allowing the resulting material to cool, feeding it into 3N-HCℓ (100 mℓ), extracting with heptane, washing the organic layer with water, washing with a saturated aqueous solution of sodium hydrogen carbonate, further three times washing with water, drying over anhydrous magnesium sulfate, distilling off the solvent under reduced pressure, distilling the residue under reduced pressure, subjecting the distillate to column chromatography and recrystallizing, to obtain the captioned compound.
Boiling point: 221 - 228°C (1 mmHg, 130 N/m²)

The following compounds are prepared in the same manner as above:
Compound No.
212. 4-(2-(4-ethylphenyl)ethenyl)-1-(1,1,2,3,3,3-hexafluoropropoxy)benzene
213. 4-(2-(4-propylphenyl)ethenyl)-1-(1,1,2,3,3,3-hexafluoropropoxy)benzene Oily substance
214. 4-(2-(4-pentylphenyl)ethenyl)-1-(1,1,2,3,3,3-hexafluoropropoxy)benzene

216. 2-chloro-4-(2-(4-ethylphenyl)ethenyl)-1-(1,1,2,3,3,3-hexafluoropropoxy)benzene
217. 4-(2-(4-ethylcyclohexyl)ethenyl)-1-(1,1,2,3,3,3-hexafluoropropoxy)benzene
218. 4-(2-(4-propylcyclohexyl)ethenyl)-1-(1,1,2,3,3,3-hexafluoropropoxy)benzene
219. 4-(2-(4-pentylcyclohexyl)ethenyl)-1-(1,1,2,3,3,3-hexafluoropropoxy)benzene

221. 2-chloro-4-(2-(4-ethylcyclohexyl)ethenyl)-1-(1,1,2,3,3,3-hexafluoropropoxy)benzene
222. 4-(2-(4-(4-ethylcyclohexyl)cyclohexyl)ethenyl)-1-(1,1,2,3,3,3-hexafluoropropoxy)benzene
223. 4-(2-(4-(4-butylcyclohexyl)cyclohexyl)ethenyl)-1-(1,1,2,3,3,3-hexafluoropropoxy)benzene
224. 4-(2-(4-(4-pentylcyclohexyl)cyclohexyl)ethenyl)-1-(1,1,2,3,3,3-hexafluoropropoxy)benzene
225. 4-(2-(4-(4-pentylcyclohexyl)cyclohexyl)ethenyl)-1-(1,1,2,3,3,3-hexafluoropropyl)benzene
226. 2-chloro-4-(2-(4-(4-ethylcyclohexyl)cyclohexyl)ethenyl)-1-(1,1,2,3,3,3-hexafluoropropoxy) benzene

229. 4-(2-(4-(4-ethylcyclohexyl)phenyl)ethenyl)-1-(1,1,2,3,3,3-hexafluoropropoxy)benzene
230. 4-(2-(4-(4-propylcyclohexyl)phenyl)ethenyl)-1-(1,1,2,3,3,3-hexafluoropropoxy)benzene
231. 4-(2-(4-(4-butylcyclohexyl)phenyl)ethenyl)-1-(1,1,2,3,3,3-hexafluordpropoxy)benzene
232. 4-(2-(4-(4-pentylcyclohexyl)phenyl)ethenyl)-1-(1,1,2,3,3,3-hexafluoropropoxy)benzene
233. 2-chloro-4-(2-(4-(4-ethylcyclohexyl)phenyl)ethenyl)-1-(1,1,2,3,3,3-hexafluoropropoxy)benzene

235. 4-(2-(2-fluoro-4-(4-ethylcyclohexyl)phenyl)ethenyl)-1-(1,1,2,3,3,3-hexafluoropropoxy)benzene
236. 4-(2-(2-fluoro-4-(4-propylcyclohexyl)phenyl)ethenyl)-1-(1,1,2,3,3,3-hexafluoropropoxy)benzene
237. 4-(2-(2-fluoro-4-(4-butylcyclohexyl)phenyl)ethenyl)-1-(1,1,2,3,3,3-hexafluoropropoxy)benzene
238. 4-(2-(2-fluoro-4-(4-pentylcyclohexyl)phenyl)ethenyl)(1,1,2,3,3,3-hexafluoropropoxy)benzene
239. 2-chloro-4-(2-(2-fluoro-4-(4-ethylcyclohexyl)phenyl)-ethenyl)-1-(1,1,2,3,3,3-hexafluoropropoxy)benzene
240. 2-fluoro-4-(2-(2-fluoro-4-(4-propylcyclohexyl)phenyl)-ethenyl)-1-(1,1,2,3,3,3-hexafluoropropoxy)benzene
241 4-(2-(4-(4-propylphenyl)phenyl)ethenyl)-1-(1,1,2,3,3,3-hexafluoropropoxy)benzene
242. 4-(2-(4-(4-pentylphenyl)phenyl)ethenyl)-1-(1,1,2,3,3,3-hexafluoropropoxy)benzene

244. 2-chloro-4-(2-(4-(4-propylphenyl)phenyl)ethenyl)-1-(1,1,2,3,3,3-hexafluoropropoxy)benzene
245. 2-methyl-4-(2-(4-(4-propylphenyl)phenyl)ethenyl-1-(1,1,2,3,3,3-hexafluoropropoxy)benzene

247. 4-(2-(4-(4-propylphenyl)cyclohexyl)ethenyl)-1-(1,1,2,3,3,3-hexafluoropropoxy)benzene
248. 4-(2-(4-(4-pentylphenyl)cyclohexyl)ethenyl)-1-(1,1,2,3,3,3-hexafluoropropoxy)benzene
249. 2-methyl-4-(2-(4-(4-propylphenyl)cyclohexyl)ethenyl)-1-(1,1,2,3,3,3-hexafluoropropoxy)benzene

253. 2-chloro-4-(2-(4-(2-fluoro-4-propylphenyl)cyclohexyl)ethenyl)-1-(1,1,2,3,3,3-hexafluoropropoxy)benzene
254. 4'-(2-(4-propylcyclohexyl)ethenyl)-4-(1,1,2,3,3,3-hexafluoropropoxy)biphenyl
255. 4'-(2-(4-butylcyclohexyl)ethenyl)-4-(1,1,2,3,3,3-hexafluoropropoxy)biphenyl
256. 4'-(2-(4-pentylcyclohexyl)ethenyl)-4-(1,1,2,3,3,3-hexafluoropropoxy)biphenyl
257. 4'-(2-(4-hexylcyclohexyl)ethenyl)-4-(1,1,2,3,3,3-hexafluoropropoxy)biphenyl
259. 4'-(2-(4-butylcyclohexyl)ethenyl)-3-chloro-4-(1,1,2,3,3,3-hexafluoropropoxy)biphenyl
261. 4'-(2-(4-hexylcyclohexyl)ethenyl)-3-methyl-4-(1,1,2,3,3,3-hexafluoropropoxy)biphenyl
262. 4'-(2-(4-(4-pentylcyclohexyl)cyclohexyl)ethenyl)-4-(1,1,2,3,3,3-hexafluoropropoxy)biphenyl

### Example 6

### Preparation of 4-(2-(4-(4-ethylcyclohexyl)phenyl)ethynyl)-1-(1,1,2,3,3,3-hexafluoropropoxy)benzene (compound No. 267)

### (a compound of the formula (1) wherein R = ethyl group; X = -C=C-; Y₁ and Y₂ =hydrogen atom; ℓ = m = 1, n = 0, p = 1; ring A = cyclohexane ring and ring B = benzene ring)

A hexane solution of n-butyllithium (6.25 mℓ, 10 mmol) (1.60 mol solution) was dropwise added at -40°C or lower, to a THF (5 mℓ) solution of 4-(4-propylcyclohexyl)phenylacetylene (2.12 g, 10 mmol), prepared according to the method of Tetrahedron Letters, 2881 (1965), followed by stirring at the same temperature for 20 minutes, adding a THF solution of zinc chloride (20 mℓ, 10 mmol) (0.5 mol solution), stirring the mixture at room temperature for 30 minutes, further adding 4-(1,1,2,3,3,3-hexafluoropropoxy)bromobenzene (3.23 g, 10 mmol) and tetrakis(triphenylphosphine)palladium (0) (500 mg), heating the mixture under reflux for 3.5 hours, allowing the resulting material to cool, feeding it into 3N-HCℓ (100 mℓ), extracting with toluene (100 mℓ), washing the organic layer with water, washing with a saturated sodium hydrogen carbonate, further three times washing with water, drying over anhydrous magnesium sulfate, distilling off the solvent under reduced pressure, subjecting the residue to column chromatography (eluent: heptane), and recrystallizing from a mixed solvent of methanol with ethanol (15 mℓ), to obtain the captioned compound (2.9 g) (yield: 64%).
Cr₁ 93.0°C Cr₂ 111.9°C SA 181.4°C N 192.3°C Iₛₒ

The following compounds are prepared in the same manner as above:
268. 4-(2-(4-propylphenyl)ethynyl)-1-(1,1,2,3,3,3-hexafluoropropoxy)benzene Cr 68.7°C Iₛₒ
269. 4-(2-(4-butylphenyl)ethynyl)-1-(1,1,2,3,3,3-hexafluoropropoxy)benzene
270. 4-(2-(4-butylphenyl)ethynyl)-1-(1,1,2,3,3,3-hexafluoropropyl)benzene
271. 4-(2-(4-pentyloxyphenyl)ethynyl)-1-(1,1,2,3,3,3-hexafluoropropoxy)benzene
272. 2-fluoro-4-(2-(4-propylphenyl)ethynyl)-1-(1,1,2,3,3,3-hexafluoropropyl)benzene

274. 4-(2-(4-propylcyclohexyl)ethynyl)-1-(1,1,2,3,3,3-hexafluoropropoxy)benzene
275. 4-(2-(4-butylcyclohexyl)ethynyl)-1-(1,1,2,3,3,3-hexafluoropropoxy)benzene
276. 4-(2-(4-pentyloxycyclohexyl)ethynyl)-1-(1,1,2,3,3,3-hexafluoropropoxy)benzene

279. 4-(2-(4-(4-propylphenyl)phenyl)ethynyl)-1-(1,1,2,3,3,3-hexafluoropropoxy)benzene
280. 4-(2-(4-(4-pentylphenyl)phenyl)ethynyl)-1-(1,1,2,3,3,3-hexafluoropropoxy)benzene
281. 4-(2-(4-(4-propoxyphenyl)phenyl)ethynyl)-1-(1,1,2,3,3,3-hexafluoropropoxy)benzene
283. 4-(2-(4-(4-propylcyclohexyl)phenyl)ethynyl)-1-(1,1,2,3,3,3-hexafluoropropoxy)benzene
285. 4-(2-(4-(4-pentylcyclohexyl)phenyl)ethynyl)-1-(1,1,2,3,3,3-hexafluoropropoxy)benzene
289. 2,6-difluoro-4-(2-(4-(4-ethylcyclohexyl)phenyl)ethynyl)-1-(1,1,2,3,3,3-hexafluoropropyl)benzene
291. 4-(2-(2-fluoro-4-(4-propoxycyclohexyl)phenyl)ethynyl)-1-(1,1,2,3,3,3-hexafluoropropoxy)benzene
293. 4-(2-(4-(4-ethylphenyl)cyclohexyl)ethynyl)-1-(1,1,2,3,3,3-hexafluoropropoxy)benzene
294. 4-(2-(4-(4-pentylphenyl)cyclohexyl)ethynyl)-1-(1,1,2,3,3,3-hexafluoropropoxy)benzene
295. 4-(2-(4-(4-propoxyphenyl)cyclohexyl)ethynyl)-1-(1,1,2,3,3,3-haxafluoropropoxy)benzene
297. 4-(2-(2-fluoro-4-(4-propoxyphenyl)cyclohexyl)ethynyl)-1-(1,1,2,3,3,3-hexafluoropropoxy)benzene (wherein fluorine atom on the cyclohexane ring is present in an axial position)
299. 4-(2-(4-(4-ethylcyclohexyl)cyclohexyl)ethynyl)-1-(1,1,2,3,3,3-hexafluoropropoxy)benzene
300. 4-(2-(4-(4-pentylcyclohexyl)cyclohexyl)ethynyl)-1-(1,1,2,3,3,3-hexafluoropropoxy)benzene
301. 4-(2-(4-(4-propoxycyclohexyl)cyclohexyl)ethynyl)-1-(1,1,2,3,3,3-hexafluoropropoxy)benzene
303. 4-(2-(2-fluoro-4-(4-propoxycyclohexyl)cyclohexyl)ethynyl)-1-(1,1,2,3,3,3-hexafluoropropoxy)benzene (wherein fluorine atom on the cyclohexane ring is present in an axial position)

305. 4'-(2-(4-propylphenyl)ethynyl)-4-(1,1,2,3,3,3-hexafluoropropoxy)biphenyl
306. 4'-(2-(4-butylphenyl)ethynyl)-4-(1,1,2,3,3,3-hexafluoropropoxy)biphenyl
307. 4'-(2-(4-pentylphenyl)ethynyl)-4-(1,1,2,3,3,3-hexafluoropropoxy)biphenyl

309. 4'-(2-(2-fluoro-4-butylphenyl)ethynyl)-4-(1,1,2,3,3,3-hexafluoropropoxy)biphenyl
310. 4-(4-(2-(4-propylphenyl)ethynyl)cyclohexyl)-1-(1,1,2,3,3,3-hexafluoropropoxy)benzene
311. 4-(4-(2-(4-butylphenyl)ethynyl)cyclohexyl)-1-(1,1,2,3,3,3-hexafluoropropoxy)benzene
312. 4-(4-(2-(4-pentylphenyl)ethynyl)cyclohexyl)-1-(1,1,2,3,3,3-hexafluoropropoxy)benzene
314. 4-(4-(2-(2-fluoro-4-butylphenyl)ethynyl)cyclohexyl)-1-(1,1,2,3,3,3-hexafluoropropoxy)benzene

### Example 7

### Preparation of 4'-(4-propylbenzyloxy)-1-(1,1,2,3,3,3-hexafluoropropoxy)biphenyl (compound No. 315) (a compound of the formula (1) wherein R₁ = propyl group; X = -CH₂O-; Y₁ and Y₂: hydrogen atom; ℓ = n = 1, m = 0, p = 1; rings A and C: benzene ring)

A THF (40 mℓ) solution of 4'-hydroxy-1-(1,1,2,3,3,3-hexafluoropropoxy)biphenyl (4.46 g, 10 mmol) prepared from commercially available bisphenol and 1,1,2,3,3,3-hexafluoroiodopropane was dropwise added to a mixture of sodium hydride (240 mg, 10 mmol) with THF (20 mℓ) under ice cooling, followed by stirring the mixture for 30 minutes, adding to the mixture, a THF (10 mℓ) solution of 4-propylbenzyl bromide (2.13 g, 10 mmol), stirring the mixture at room temperature for 3 hours, feeding the resulting material into 3N-HCℓ, extracting with toluene (100 mℓ), washing with water, then with a saturated aqueous solution of sodium hydrogen carbonate, further three times washing with water, drying over anhydrous magnesium sulphate, distilling off the solvent under reduced pressure, distilling the residue under reduced pressure, recrystallizing the distillate under reduced pressure, subjecting it to column chromatography (eluent: heptane), and recrystallizing from ethanol to obtain the captioned compound.
Boiling point: 260 - 265°C (1 mmHg, 130 N/m²)

The following compounds are prepared in the same manner as above:
Compound No.
316. 4-(ethylbenzyloxy)-1-(1,1,2,3,3,3-hexafluoropropoxy)benzene
318. 4-(4-propylcyclohexylmethyloxy)-1-(1,1,2,3,3,3-hexafluoropropoxy)benzene
320. 4'-(4-ethylbenzyloxy)-1-(1,1,2,3,3,3-hexatluoropropoxy)biphenyl
321. 4'-(4-ethylbenzyloxy)-2-fluoro-1-(1,1,2,3,3,3-hexafluoropropoxy)biphenyl
322. 4'-(4-ethylcyclohexylmethyloxy)-1-(1,1,2,3,3,3-hexafluoropropoxy)biphenyl

325. 4'-(4-propylcyclohexylmethyloxy)-2,6-difluoro-1-(1,1,2,3,3,3-hexafluoropropyl)biphenyl
326. 4-(4-(4-propylcyclohexyl)cyclohexylmethyloxy)-1-(1,1,2,3,3,3-hexafluoropropoxy)benzene

### Example 8

### Preparation of 4-(4-(1,1,2,3,3,3-hexafluoropropoxy)benzyloxy)-1-(4-pentylcyclohexyl)benzene (compound No. 328)

### (a compound of the formula (1) wherein R₁ = pentyl group; X = -OCH₂-; Y₁ and Y₂ hydrogen atom; ℓ=m=1, n=0, p = 1; ring A = cyclohexyl ring; and ring B = benzene ring)

A THF (30 mℓ) solution of 4-(4-propylcyclohexyl)phenol (2.19 g, 10 mmol) prepared according to Japanese patent application laid-open Nos. Sho 57-64626 and Sho 57-165328, was dropwise added to a mixture of sodium hydride (240 mg, 10 mmol) with THF (20 mℓ). stirring the mixture under ice-cooling for 30 minutes, followed by adding a THF (10 mℓ) solution of 4-(1,1,2,3,3,3-hexafluoropropoxy)benzyl chloride (2.92 g, 10 mmol), prepared from commercially available 4-hydroxybenzyl chloride and 1,1,2,3,3,3-hexafluoroiodopropane, stirring the mixture at room temperature for 3 hours. feeding the resulting material into 3N-HCℓ, extracting with toluene (100 mℓ), washing with water, washing with a saturated aqueous solution of sodium hydrogen carbonate, further three times washing with water, drying over anhydrous magnesium sulfate, distilling off the solvent under reduced pressure, distilling the residue under reduced pressure, subjecting the distillate to column chromatography (eluent: heptane) and recrystallizing from ethanol, to obtain the captioned compound.
Boiling point: 238 - 244°C (1 mmHg, 130 N/m²)

The following compounds are prepared in the same manner as above:
Compound No.
329. 4-(4-(1,1,2,3,3,3-hexafluoropropoxy)benzyloxy)propylbenzene
331. 4-(4-(1,1,2,3,3,3-hexafluoropropoxy)benzyloxy)-3-fluoro-1-pentylbenzene
333. 4-(4-(1,1,2,3,3,3-hexafluoropropoxy)benzyloxy)-1-propylcyclohexane
336. 4-(4-(1,1,2,3,3,3-hexafluoropropoxy)benzyloxy)-1-(4-(4-propylcyclohexyl)phenyl)benzene
337. 4-(4-(1,1,2,3,3,3-hexafluoropropoxy)benzyloxy)-1-(4-(4-propylphenyl)cyclohexyl)benzene
338. 4-(4-(1,1,2,3,3,3-hexafluoropropoxy)phenyl)cyclohexylmethyloxy-1-propylcyclohexane
340. 4-(4-(1,1,2,3,3,3-hexafluoropropoxy)phenyl)benzyloxy-1-propylcyclohexane
343. 4-(3,5-difluoro-4-(1,1,2,3,3,3-hexafluoropropyl)phenyl)benzyloxy-1-propylcyclohexane

### Example 9

### Preparation of 4'-(1,1,2,3,3,3-hexafluoropropoxy)phenyl)biphenylyl 4-propylcyclohexylcarboxylate (compound No. 344)

### (a compound of the formula (1) wherein R₁ = propyl group; X -CO-O-; Y₁ and Y₂ = hydrogen atom; ℓ = n = 1, m = 0, p = 1; and ring A = cyclohexane ring and ring C = benzene ring)

Dicyclohexylcarbodiimide (2.06 g, 10 mmol) and dimethylaminopyridine (122 mg, 1 mmol) were added to a mixture of 4'-hydroxy-1-(1,1,2,3,3,3-hexafluoropropoxy)biphenyl (4.46 g, 10 mmol) prepared in Example 7, 4-propylcyclohexanecarboxylic acid (1.70 g, 10 mmol) and dichloromethane (30 mℓ), followed by stirring the resulting mixture at room temperature overnight, filtering off the insolubles, distilling off the solvent under reduced pressure, subjecting the residue to column chromatography (eluent: heptane) and recrystallizing from ethanol, to obtain the captioned compound.

The following compounds are obtained in the same manner as above.
Compound No.
345. 4'-(1,1,2,3,3,3-hexafluoropropoxy)phenyl)biphenylyl 4-pentylcyclohexylcarboxylate
348. 4-(1,1,2,3,3,3-hexafluoropropoxy)phenyl 4-propylcyclohexylcarboxylate Oily substance
349. 4-(1,1,2,3,3,3-hexafluoropropoxy)phenyl 4-pentylcyclohexylcarboxylate
352. 3-fluoro-4-(1,1,2,3,3,3-hexafluoropropyl)phenyl 4-pentylcyclohexylcarboxylate

### Example 10

### Preparation of 4-(4-propylcyclohexyl)phenyl 4-(1,1,2,3,3,3-hexafluoropropoxy)benzoate (compound No. 353)

### (a compound of the formula (I), wherein R₁ =propyl group; X = -O-CO-; Y₁ and Y₂ hydrogen atom; ℓ=m=1, n=0,p= 1; and ring A = cyclohexane ring; and ring B = benzene ring)

A mixture of 4-(4-propylcyclohexyl)phenol prepared in Example 8, 4-(4-propylcyclohexyl)phenyl 4-hydroxybenzoate (3.39 g. 10 mmol) prepared from 4-hydroxybenzoic acid according to the method of Tetrahydrogen Letters, 3453 (1971) and 1,1,2,3,3,3-hexafluoroiodopropane (5.50 g, 20 mmol), was stirred at 60°C, under 10 atm, for 3 hours, followed by allowing the resulting material to cool, distilling off the solvent, subjecting the residue to column chromatography (eluent: haptane) and recrystallizing from ethanol, to obtain the captioned compound.

The following compounds are prepared in the same manner as above:
Compound No.
354. 4-(4-pentylcyclohexyl)phenyl 4-(1,1,2,3,3,3-hexafluoropropoxy)benzoate
357. 4-(4-propylcyclohexyl)phenyl 3-fluoro-4-(1,1,2,3,3,3-hexafluoropropyl)benzoate
359. 4-propylcyclohexyl 4-(1,1,2,3,3,3-hexafluoropropoxy)benzoate
360. 4-pentylcyclohexyl 4-(1,1,2,3,3,3-hexafluoropropoxy)benzoate

362. 4-propylphenyl 4-(1,1,2,3,3,3-hexafluoropropoxy)benzoate
363. 4-propylphenyl 4-(1,1,2,3,3,3-hexafluoropropyl)benzoate
364. 4-pentylphenyl 4-(1,1,2,3,3,3-hexafluoropropoxy)benzoate
365. 4-propylphenyl 3-fluoro-4-(1,1,2,3,3,3-hexafluoropropoxy)benzoate

367. 4-(4-propylcyclohexyl)cyclohexyl 4-(1,1,2,3,3,3-hexafluoropropoxy)benzoate
368. 4-(4-pentylcyclohexyl)cyclohexyl 4-(1,1,2,3,3,3-hexafluoropropoxy)benzoate
369. 4-(4-propylcyclohexyl)cyclohexyl 3-chloro-4-(1,1,2,3,3,3-hexafluoropropoxy)benzoate

371. 4-propylcyclohexyl 4-(4-(1,1,2,3,3,3-hexafluoropropoxy)phenyl)benzoate
372. 4-pentylcyclohexyl 4-(4-(1,1,2,3,3,3-hexafluoropropoxy)phenyl)benzoate

### Example 11

### Preparation of 4'-(4-methoxymethylcyclohexyl)-3-fluoro-4-(1,1,2,3,3,3-hexafluoropropyloxy)biphenyl

### (a compound of the formula (I) wherein R₁ = methoxymethyl group; X = covalent bond; Y₁ =hydrogen atom; Y₂ = fluorine atom; = = m = 1, n = 0, p = 1; ring A = cyclohexane ring; and ring B = benzene ring)

A hexane solution of n-butyllithium (12.3 mℓ, 20 mmol) (1.63 mol solution) was added to a THF (60 mℓ) solution of 4-(4-methoxymethylcyclohexyl)-1-iodobenzene (5.51 g, 17 mmol) at -50°C in a nitrogen atmosphere, followed by stirring the mixture at the same temperature for 90 minutes, adding a THF solution (0.5 mol) of zinc chloride (40 mℓ, 20 mmol), stirring the mixture at room temperature for one hour, further adding tetrakis(triphenylphosphine)palladium (0) (0.5 g) and 4-(1,1,2,3,3,3-hexafluoropropoxy)-3-fluorobromobenzene (6.82 g, 20 mmol), heating the mixture under reflux for 5 hours, allowing the resulting material to cool, adding toluene (200 mℓ) to the resulting reaction mixture, washing the organic layer successively with 3N-HCℓ (200 mℓ), water (200 mℓ) and a saturated aqueous solution of sodium hydrogen carbonate (200 mℓ), next three times washing with water (200 mℓ), drying over anhydrous magnesium sulfate, distilling off the solvent under reduced pressure, subjecting the residue to column chromatography (eluent: heptane/toluene = 1:1), and recrystallizing from heptane, to obtain the captioned compound (1.47 g) (yield: 16%).
Cr 86.4 SA 134.2 N 143.2 Iₛₒ

The following compounds are prepared in the same manner as above:
Compound No.
376. 4-(4-methoxymethylcyclohexyl)-1-(1,1,2,3,3,3-hexafluoropropyloxy)benzene
377. 4'-(4-methoxymethylcyclohexyl)-4-(1,1,2,3,3,3-hexafluoropropyloxy)biphenyl
378. 4'-(4-ethoxymethylcyclohexyl)-4-(1,1,2,3,3,3-hexafluoropropyloxy)biphenyl
379. 4'-(4-propoxymethylcyclohexyl)-4-(1,1,2,3,3,3-hexafluoropropyloxy)biphenyl
380. 4'-(4-methoxyethylcyclohexyl)-4-(1,1,2,3,3,3-hexafluoropropyloxy)biphenyl
381. 4'-(4-ethoxyethylcyclohexyl)-4- (1,1,2,3,3,3-hexafluoropropyloxy)biphenyl

392. 4-(4-methoxymethoxycyclohexyl)-1-(1,1,2,3,3,3-hexafluoropropyloxy)benzene
393. 4'-(4-methoxymethoxycyclohexyl)-4-(1,1,2,3,3,3-hexafluoropropyloxy)biphenyl
394. 4'-(4-ethoxymethoxycyclohexyl)-4-(1,1,2,3,3,3-hexafluoropropyloxy)biphenyl
395. 4'-(4-methoxyethoxycyclohexyl)-4-(1,1,2,3,3,3-hexafluoropropyloxy)biphenyl

401. 4'-(4-methoxymethylcyclohexyl)-4-(1,1,2,3,3,3-hexafluoropropyl)biphenyl
402. 4'-(4-ethoxymethylcyclohexyl)-4-(1,1,2,3,3,3-hexafluoropropyl)biphenyl
403. 4'-(4-methoxymethylcyclohexyl)-3-fluoro-4-(1,1,2,3,3,3-hexafluoropropyl)biphenyl
404. 4'-(4-ethoxymethylcyclohexyl)-3-fluoro-4-(1,1,2,3,3,3-hexafluoropropyl)biphenyl
405. 4'-(4-methoxymethylcyclohexyl)-3,5-difluoro-4-(1,1,2,3,3,3-hexafluoropropyl)biphenyl
406. 4'-(4-ethoxymethylcyclohexyl)-3,5-difluoro-4-(1,1,2,3,3,3-hexafluoropropyl)biphenyl
407. 4'-(4-methoxymethoxycyclohexyl)-4-(1,1,2,3,3,3-hexafluoropropyl)biphenyl
408. 4'-(4-ethoxymethoxycyclohexyl)-4-(1,1,2,3,3,3-hexafluoropropyl)biphenyl
409. 4'-(4-methoxymethoxycyclohexyl)-3-fluoro-4-(1,1,2,3,3,3-hexafluoropropyl)biphenyl
410. 4'-(4-ethoxymethoxycyclohexyl)-3-fluoro-4-(1,1,2,3,3,3-hexafluoropropyl)biphenyl
411. 4'-(4-methoxymethoxycyclohexyl)-3,5-difluoro-4-(1,1,2,3,3,3-hexafluoropropyl)biphenyl
412. 4'-(4-ethoxymethoxycyclohexyl)-3,5-difluoro-4-(1,1,2,3,3,3-hexafluoropropyl)biphenyl

In the same manner as in Example 5, Example 6 and Example 9, the following compounds of compound Nos. 413-428, 429-433, 434-449, 450-461 and 462-485, can be prepared:
Compound No.
413. 4-(4-(4-methoxymethylcyclohexyl)cyclohexyl)-1-(1,1,2,3,3,3-hexafluoropropyloxy)benzene
414. 4-(4-(4-ethoxymethylcyclohexyl)cyclohexyl)-1-(1,1,2,3,3,3-hexafluoropropyloxy)benzene
415. 4-(4-(4-methoxymethylcyclohexyl)cyclohexyl)-1-(1,1,2,3,3,3-hexafluoropropyloxy)-2-fluorobenzene

417. 4-(4-(4-methoxymethoxycyclohexyl)cyclohexyl)-1-(1,1,2,3,3,3-hexafluoropropyloxy)benzene
418. 4-(4-(4-ethoxymethoxycyclohexyl)cyclohexyl)-1-(1,1,2,3,3,3-hexafluoropropyloxy)benzene

421. 4-(4-(4-methoxymethylcyclohexyl)cyclohexyl)-1-(1,1,2,3,3,3-hexafluoropropyl)benzene
422. 4-(4-(4-ethoxymethylcyclohexyl)cyclohexyl)-1-(1,1,2,3,3,3-hexafluoropropyl)benzene
423. 4-(4-(4-methoxymethylcyclohexyl)cyclohexyl)-1-(1,1,2,3,3,3-hexafluoropropyl)-2-fluorobenzene
424. 4-(4-(4-methoxymethylcyclohexyl)cyclohexyl)-1-(1,1,2,3,3,3-hexafluoropropyl)-2,4-difluorobenzene
425. 4-(4-(4-methoxymethoxycyclohexyl)cyclohexyl)-1-(1,1,2,3,3,3-hexafluoropropyl)benzene
426. 4-(4-(4-ethoxymethoxycyclohexyl)cyclohexyl)-1-(1,1,2,3,3,3-hexafluoropropyl)benzene
427. 4-(4-(4-methoxymethoxycyclohexyl)cyclohexyl)-1-(1,1,2,3,3,3-hexafluoropropyl)-2-fluorobenzene
428. 4-(4-(4-methoxymethoxycyclohexyl)cyclohexyl)-1-(1,1,2,3,3,3-hexafluoropropyl)-2,4-difluorobenzene
429. 4-(2-(4-(4-methoxymethylcyclohexyl)cyclohexyl)ethyl-1-(1,1,2,3,3,3-hexafluoropropyloxy)benzene
430. 4-(2-(4-(4-methoxymethylcyclohexyl)cyclohexyl)ethyl)-1-(1,1,2,3,3,3-hexafluoropropyl)benzene
431. 4-(2-(4-(4-methoxymethoxycyclohexyl)cyclohexyl)ethyl)-1-(1,1,2,3,3,3-hexafluoropropyloxy)benzene

434. 4'-(2-(4-(4-methoxymethylcyclohexyl)ethyl)-4-(1,1,2,3,3,3-hexafluoropropyloxy)biphenyl
435. 4'-(2-(4-ethoxymethylcyclohexyl)ethyl)-4-(1,1,2,3,3,3-hexafluoropropyloxy)biphenyl

437. 2',6'-difluoro-4'-(2-(4-methoxymethylcyclohexyl)ethyl)-3,5-difluoro-4-(1,1,2,3,3,3-hexafluoropropyloxy)biphenyl
438. 4'-(2-(4-methoxymethoxycyclohexyl)ethyl)-4-(1,1,2,3,3,3-hexafluoropropyloxy)biphenyl
439. 4'-(2-(4-ethoxymethoxycyclohexyl)ethyl)-4-(1,1,2,3,3,3-hexafluoropropyloxy)bipheny)

442. 4'-(2-(4-methoxymethylcyclohexyl)ethyl)-4-(1,1,2,3,3,3-hexafluoropropyl)biphenyl
443. 4'-(2-(4-ethoxymethylcyclohexyl)ethyl)-4-(1,1,2,3,3,3-hexafluoropropyl)biphenyl
444. 4'-(2-(4-methoxymethylcyclohexyl)ethyl)-3,5-diflroro-4-(1,1,2,3,3,3-hexafluoropropyl)biphenyl
445. 2',6'-difluoro-4'-(2-(4-methoxymethylcyclohexyl)ethyl)-3,5-difluoro-4-(1,1,2,3,3,3-hexafluoropropyl)biphenyl
446. 4'-(2-(4-methoxymethoxycyclohexyl)ethyl)-4-(1,1,2,3,3,3-hexafluoropropyl)biphenyl
447. 4'-(2-(4-ethoxymethoxycyclohexyl)ethyl)-4-(1,1,2,3,3,3-hexafluoropropyl)biphenyl
448. 4'-(2-(4-methoxymethoxycyclohexyl)ethyl)-3,5-difluoro-4-(1,1,2,3,3,3-hexafluoropropyl)biphenyl
449. 2',6'-difluoro-4'-(2-(4-methoxymethoxycyclohexyl)ethyl)-3,5-difluoro-4-(1,1,2,3,3,3-hexafluoropropyl)biphenyl
450. 4-(2-(4-(4-methoxymethylcyclohexyl)cyclohexyl)ethenyl)-1-(1,1,2,3,3,3-hexafluoropropyloxy)benzene
451. 4-(2-(4-(4-methoxymethoxycyclohexyl)cyclohexyl)ethenyl)-1-(1,1,2,3,3,3-hexafluoropropyloxy)benzene

454. 4-(2-(4-(4-methoxymethylcyclohexyl)cyclohexyl)ethenyl)-1-(1,1,2,3,3,3-hexafluoropropyl)benzene
455. 4-(2-(4-(4-methoxymethyloxycyclohexyl)cyclohexyl)ethenyl)-1-(1,1,2,3,3,3-hexafluoropropyl)-2,6-difluorobenzene
456. 4-(2-(4-(4-methoxymethylcyclohexyl)cyclohexyl)ethynyl)-1-(1,1,2,3,3,3-hexafluoropropyloxy)benzene
457. 4-(2-(4-(4-methoxymethoxycyclohexyl)cyclohexyl)ethynyl)-1-(1,1,2,3,3,3-herafluoropropyloxy)benzene

460. 4-(2-(4-(4-methoxymethylcyclohexyl)cyclohexyl)ethynyl)-1-(1,1,2,3,3,3-hexafluoropropyl)benzene
461. 4-(2-(4-(4-methoxymethyloxycyclohexyl)cyclohexyl)ethynyl)-1-(1,1,2,3,3,3-hexafluoropropyl)-2,6-difluorobenzene
462. 4-(1,1,2,3,3,3-hexafluoropropyloxy)phenyl 4-methoxymethylcyclohexylcarboxylate
463. 4-(1,1,2,3,3,3-hexafluoropropyloxy)phenyl 4-ethoxymethylcyclohexylcarboxylate
464. 4-(1,1,2,3,3,3-hexafluoropropyloxy)phenyl 4-methoxymethoxycyclohexylcarboxylate
465. 4-(1,1,2,3,3,3-hexafluoropropyloxy)phenyl 4-ethoxymethoxycyclohexylcarboxylate
466. 4-(1,1,2,3,3,3-hexafluoropropyloxy)phenyl 4-methoxyethoxycyclohexylcarboxylate
467. 4-(1,1,2,3,3,3-hexafluoropropyloxy)phenyl 4-ethoxyethoxycyclohexylcarboxylate

470. 4-(1,1,2,3,3,3-hexatluoropropyloxy)biphenylyl 4-methoxymethylcyclohexylcarboxylate
471. 4-(1,1,2,3,3,3-hexafluoropropyloxy)biphenyl 4-ethoxymethylcyclohexylcarboxylate
472. 4-(1,1,2,3,3,3-hexafluoropropyloxy)biphenyl 4-methoxymethoxycyclohexylcarboxylate
473. 4-(1,1,2,3,3,3-hexafluoropropyloxy)biphenyl 4-ethoxymethoxycyclohexylcarboxylate
474. 4-(1,1,2,3,3,3-hexafluoropropyloxy)biphenyl 4-methoxyethoxycyclohexylcarboxylate
475. 4-(1,1,2,3,3,3-hexafluoropropyloxy)biphenyl 4-ethoxyethoxycyclohexylcarboxylate

478. 4-(1,1,2,3,3,3-hexafluoropropyl)phenyl 4-methoxymethylcyclohexylcarboxylate
479. 4-(1,1,2,3,3,3-hexafluoropropyl)phenyl 4-ethoxymethylcyclohexylcarboxylate
480. 4-(1,1,2,3,3,3-hexafluoropropyl)phenyl 4-methoxymethoxycyclohexylcarboxylate
481. 4-(1,1,2,3,3,3-hexafluoropropyl)phenyl 4-ethoxymethoxycyclohexylcarboxylate
482. 4-(1,1,2,3,3,3-hexafluoropropyl)phenyl 4-methoxyethoxycyclohexylcarboxylate
483. 4-(1,1,2,3,3,3-hexafluoropropyl)phenyl 4-ethoxyethoxycyclohexylcarboxylate
484. 3-fluoro-4-(1,1,2,3,3,3-hexafluoropropyl)phenyl 4-methoxymethylcyclohexylcarboxylate
485. 3,5-difluoro-4-(1,1,2,3,3,3-hexafluoropropyl)phenyl 4-methoxymethylcyclohexylcarboxylate

### Example 12 (Use example 1)

A lqiuid crystal composition A consisting of the following composition was prepared as a base mixture:

| | |
|---|---|
| 1,2-difluoro-4-(4-(4-ethylcyclohexyl)cyclohexyl)benzene | 9.09% |
| 1,2-difluoro-4-(4-(4-propylcyclohexyl)cyclohexyl)benzene | 9.09% |
| 1,2-difluoro-4-(4-(4-pentylcyclohexyl)cyclohexyl)benzene | 9.09% |
| 1,2-difluoro-4-(4-(2-(4-ethylcyclohexyl)ethyl)cyclohexyl)benzene | 18.18% |
| 1,2-difluoro-4-(4-(2-(4-propylcyclohexyl)ethyl)cyclohexyl)benzene | 9.09% |
| 1,2-difluoro-4-(4-(2-(4-pentylcyclohexyl)ethyl)cyclohexyl)benzene | 18.18% |
| 3,4-difluoro-4'-(4-ethylcyclohexyl)biphenyl | 9.09% |
| 3,4-difluoro-4'-(4-propylcyclohexyl)biphenyl | 9.09% |
| 3,4-difluoro-4'-(4-pentylcyclohexyl)biphenyl | 9.09% |

The values of the physical properties of this composition were as follows:
Clearing point: 100.2°C, Δn: 0.093, Δc:5.1, viscosity η at 20° C: 24.5 cP, and threshold voltage in a cell thickness of 8 µm: 2.15V.

Next, 4'-(1,1,2,3,3,3-hexafluoropropoxy)-4-(4-propylcyclohexyl)biphenyl (compound No. 1) (20%) was added to the above composition A (80%) to prepare a composition B₁, followed by determining its values of physical properties. The results were as follows (the values within the parenthese refer to calculated extraporated values; this applies to those of the following Examples):
Clearing point: 109.1°C (144.7°C), Δn: 0.103 (0.143), △ε: 4.9, threshold voltage in a cell thickness of 8.8 µm: 2.35V.

### Example 13 (Use exmaple 2) (for comparison purposes)

Example 11 was repeated except that compound No. 1 was replaced by 4'-(4-propylcyclohexyl)-3-fluoro-4-(1,1,2,3,3,3-hexafluoropropoxy)biphenyl (compound No. 22), to prepare composition B₂. The values of physical properties of this composition were as follows:
Clearing point: 106.1°C (129.7°C), Δn: 0.102 (0.138) △ε: 5.2, viscosity at 20°C, η:26.9 cP, and threshold voltage in a cell thickness of 8.8 µm: 2.28 V.

### Example 14 (Use example 3) (for comparison purposes)

Example 11 was repeated except compound No. 1 was replaced by 4'-(4-propylcyclohexyl)-3,5-difluoro-4-(1,1,2,3,3,3-hexafluoropropoxy)biphenyl (compound No. 28), to prepare a composition B₃. The values of physical properties of this composition B₃ were as follows:
Clearing point: 102.8°C (113.2°C), Δn: 0.101 (0.133), Δε:5.6, viscosity η at 20°C: 28.4 cP; and threshold voltage in a cell thickness of 8.7 µm: 2.11 V.

### Example 15 (Use example 4)

Example 11 was repeated except that compound No. 1 was replaced by 2'-fluoro-4'-(4-propylcyclohexyl)-4-(1,1,2,3,3,3-hexafluoropropoxy)biphenyl (compound No. 34), to prepare a composition B₄. The values of physical properties of this composition B₄ were as follows:
Clearing point: 102.8°C (113.2°C). Δn: 0.100 (0.129), △ε: 5.0 (4.6), viscosity at 20°C η: 27.4 (39.1) cP, and threshold voltage in a cell thickness of 8.7 µm: 2.26 V.

### Example 16 (Use example 5) ( for comparison purposes)

Example 11 was repeated except that compound No. 1 was replaced by 2,6-difluoro-4-(2-(4-(4-propylcyclohexyl)phenyl)ethynyl)-1-(1,1,2,3,3,3-hexafluoropropoxy)benzene to prepare a composition B₅. The values of physical properties of this composition B₅ were as follows:
Clearing point: 106.5°C (131.7°C), Δn: 0.112 (0.187). Δε: 5.9 (9.1), viscosity at 20°C, η: 29.9 (51.6) cP, and threshold voltage in a cell thickness of 8.7 µm: 2.11 V.

### Example 17 (Use example 6)

A liquid crystal composition C consisting of the following composition was prepared as a base mixture:

| | |
|---|---|
| 4-(4-propylcyclohexyl)benzonitrile | 30% |
| 4-(4-pentylcyclohexyl)benzonitrile | 40% |
| 4-(4-heptylcyclohexyl)benzonitrile | 30%. |

The values of physical properties of this composition C were as follows:
Clearing point: 52.3°C, △n: 0.119, △c: 10.7, viscosity at 20°C η: 21.7 m Pas, and threshold voltage in a cell thickness of 9.7 µm: 1.60V.

Next, 4'-(4-methoxymethylcyclohexyl)-3-fluoro-4-(1,1,2,3,3,3-hexafluoropropyloxy)biphenyl (20%) was added to the composition C (80%), to prepare a composition B₆, followed by determining its values of physical properties. The values were as follows (the values inside the parenthes refer to extrapolated values):
Clearing point: 56.3°C (79.0°C), Δn: 0.121 (0.130), Δε: 10.5 (9.4), viscosity at 20°C η: 27.1 (57.6)m Pas, and threshold voltage in a cell thickness of 8.7 µm: 1.51 V.

### Effectiveness of the Invention

As shown in Examples, the compounds provided by the present invention have the following characteristics:
1) a large Δn,
2) a low viscosity,
3) a high chemical stability and a high VHR,
4) since the clearing point and the compativility are very high, reduction in the nematic liquid phase of the composition does not occur, and
5) a very high Δε.

Thus, when the compound of the present invention is used as a component of a liquid crystal composition, it is possible to provide a liquid crystal composition having a large Δn, a low viscosity and a high voltage retention and exhibiting a nematic liquid crystal phase within a broad temperature range. In particular, it is possible to provide a liquid crystal composition suitable to use for TFT, since it has a very high △ε.

## Claims

1. A liquid crystalline compound expressed by the formula (1) 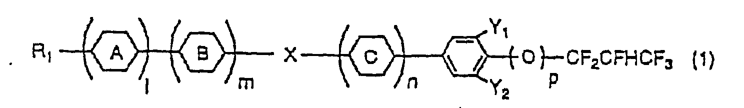 wherein R₁ represents an alkyl group of 1 to 12 carbon atoms and one CH₂ group or two CH₂ groups not adjacent to each other in said alkyl group may be replaced by group(s) selected from among oxygen atom, -CO-group, -O-CO-group, -CO-O-group and -CH=CH-group; X represents a covalent bond, -CH₂CH₂-, -CH=CH-, -C≡C-, -CH₂O-, -OCH₂-, -CO-O- or -O-CO-; Y₁ and Y₂ each independently represent hydrogen atom, fluorine atom, chlorine atom or methyl group; rings A, B and C each independently represent a benzene ring or a cyclohexane ring and these rings may be substituted by halogen atom(s) or methyl group(s); and ℓ, m, n and p each independently represent 1 or 0, but ℓ+m is one or more, with the proviso
that when p = 1, then weither Y₁ nor Y₂ represent a fluorine atom.

2. A liquid crystalline compound according to claim 1, wherein R₁ represents an alkyl group of 1 to 12 carbon atoms and said one CH₂ group or two CH₂ groups not adjacent to each other in said alkyl group may be replaced by oxygen atom or -CH=CH- group; X represents a covelent bond, -CH₂CH₂- or -C≡C-; and Y₁ and Y₂ each independently represent hydrogen atom or fluorine atom.

3. A liquid crystalline compound according to claim 2, wherein said R₁ represents an alkyl group of 1 to 12 carbon atoms.

4. A liquid crystalline compound according to claim 3, wherein said ring C represents a benzene ring or benzene ring substituted by halogen atom or methyl group.

5. A liquid crystalline compound according to claim 3, wherein said ring C represents a cyclohexane ring or a cyclohexane ring substituted by halogen atom or methyl group.

6. A liquid crystalline compound according to claim 2, wherein said R₁ represents an alkyl group of 1 to 12 carbon atoms and said one CH₂ group or two CH₂ groups not adjacent to each other in said alkyl group is replaced by oxygen atom.

7. A liquid crystalline compound according to claim 6, wherein said ring C represents benzene ring or a benzene ring substituted by halogen atom or methyl group.

8. A liquid crystalline compound according to claim 6, wherein said ring C represents cyclohexane ring or a cyclohexane ring substituted by halogen atom or methyl group.

9. A liquid crystal composition comprising at least two components at least one of which is a liquid crystalline compound set forth in any one of claims 1 to 8.

## Patentansprüche

1. Flüssigkristallverbindung, dargestellt durch die Formel (1) 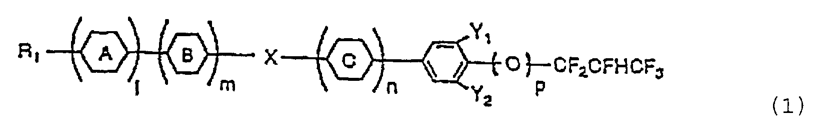 wobei R₁ eine Alkylgruppe mit 1 bis 12 Kohlenstoffatomen darstellt, und eine CH₂ Gruppe oder zwei CH₂ Gruppen, nicht benachbart zueinander in der Alkylgruppe, durch Gruppe(n) ausgewählt aus Sauerstoffatom, -CO-Gruppe, -O-CO-Gruppe, -CO-O-Gruppe und -CH=CH-Gruppe ersetzt sein können; X eine kovalente Bindung, -CH₂CH₂-, -CH=CH-, -C≡C-, -CH₂O-, -OCH₂-, -CO-O- oder -O-CO- darstellt; Y₁ und Y₂ jeweils unabhängig Wasserstoffatom, Fluoratom, Chloratom oder Methylgruppe darstellen; Ringe A, B und C jeweils unabhängig einen Benzolring oder einen Cyclohexanring darstellen, und diese Ringe durch Halogenatom(e) oder Methylgruppe(n) substituiert sein können; und 1, m, n und p jeweils unabhängig 1 oder 0 sind, aber 1+m eins oder mehr ist, mit dem Vorbehalt, dass wenn p=1 weder Y₁ noch Y₂ Fluoratom sind.

2. Flüssigkristallverbindung nach Anspruch 1, wobei R₁ eine Alkylgruppe mit 1 bis 12 Kohlenstoffatomen darstellt, und die eine CH₂ Gruppe oder zwei CH₂ Gruppen, nicht benachbart zueinander in der Alkylgruppe, durch Sauerstoffatom oder-CH=CH-Gruppe ersetzt sein können; X eine kovalente Bindung,-CH₂CH₂- oder -C≡C- darstellt; und Y₁ und Y₂ jeweils unabhängig Wasserstoffatom oder Fluoratom darstellen.

3. Flüssigkristallverbindung nach Anspruch 2, wobei R₁ eine Alkylgruppe mit 1 bis 12 Kohlenstoffatomen darstellt.

4. Flüssigkristallverbindung nach Anspruch 3, wobei der Ring C einen Benzolring oder durch Halogenatom oder Methylgruppe substituierten Benzolring darstellt.

5. Flüssigkristallverbindung nach Anspruch 3, wobei der Ring C einen Cyclohexanring oder einen durch Halogenatom oder Methylgruppe substituierten Cyclohexanring darstellt.

6. Flüssigkristallverbindung nach Anspruch 2, wobei R₁ eine Alkylgruppe mit 1 bis 12 Kohlenstoffatomen darstellt, und die eine CH₂ Gruppe oder zwei CH₂ Gruppen, nicht benachbart zueinander in der Alkylgruppe, durch Sauerstoffatom ersetzt ist.

7. Flüssigkristallverbindung nach Anspruch 6, wobei der Ring C einen Benzolring oder einen durch Halogenatom oder Methylgruppe substituierten Benzolring darstellt.

8. Flüssigkristallverbindung nach Anspruch 6, wobei der Ring C einen Cyclohexanring oder einen durch Halogenatom oder Methylgruppe substituierten Cyclohexanring darstellt.

9. Flüssigkristallzusammensetzung, enthaltend mindestens zwei Komponenten, von denen zumindest eine eine Flüssigkristallverbindung, wie in einem der vorhergehenden Ansprüche 1-8 dargelegt ist.

## Revendications

1. Composé cristallin liquide représenté par la formule (1) 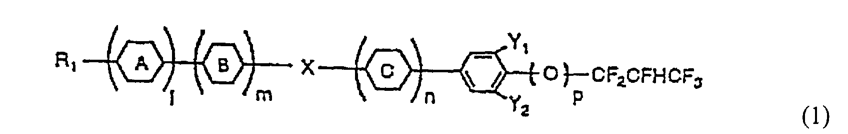 dans laquelle R₁ représente un groupe alkyle de 1 à 12 atomes de carbone, et un groupe CH₂ ou deux groupes CH₂ non adjacents l'un à l'autre dans ledit groupe alkyle peut (peuvent) être remplacé(s) par un (des) groupe(s) choisis parmi un atome d'oxygène, le groupe -CO-, le groupe -O-CO-, le groupe -CO-O- et le groupe -CH=CH-; X représente une liaison covalente, -CH₂CH₂-, -CH=CH-, -C≡C-, -CH₂O-, -OCH₂-, -CO-O- ou -O-CO-; Y₁ et Y₂ représentent indépendamment un atome d'hydrogène, un atome de fluor, un atome de chlore ou un groupe méthyle; les cycles A, B et C représentent chacun indépendamment un cycle benzénique ou un cycle cyclohexane et ces cycles peuvent être substitués par un (des) atome(s) d'halogène ou un (des) groupe(s) méthyle; et ℓ, m, n et p représentent chacun indépendamment 1 ou 0, mais ℓ+m est un ou plus, sous réserve que
lorsque p=1, alors ni Y₁ ni Y₂ ne représentent un atome de fluor.

2. Composé cristallin liquide selon la revendication 1, dans lequel R₁ représente un groupe alkyle de 1 à 12 atomes de carbone et lesdits un groupe CH₂ ou deux groupes CH₂ non adjacents l'un à l'autre dans ledit groupe alkyle peut (peuvent) être remplacé(s) par un atome d'oxygène ou un groupe -CH=CH-; X représente une liaison covalente, -CH₂CH₂- ou -C≡C-; et Y₁ et Y₂ représentent chacun indépendamment un atome d'hydrogène ou un atome de fluor.

3. Composé cristallin liquide selon la revendication 2, dans lequel ledit R₁ représente un groupe alkyle de 1 à 12 atomes de carbone.

4. Composé cristallin liquide selon la revendication 3, dans lequel ledit cycle C représente un cycle benzénique ou un cycle benzénique substitué par un atome d'halogène ou un groupe méthyle.

5. Composé cristallin liquide selon la revendication 3, dans lequel ledit cycle C représente un cycle cyclohexane ou un cycle cyclohexane substitué par un atome d'halogène ou un groupe méthyle.

6. Composé cristallin liquide selon la revendication 2, dans lequel ledit R₁ représente un groupe alkyle de 1 à 12 atomes de carbone et lesdits un groupe CH₂ ou deux groupes CH₂ non adjacents l'un à l'autre dans ledit groupe alkyle est (sont) remplacé(s) par un atome d'oxygène.

7. Composé cristallin liquide selon la revendication 6, dans lequel ledit cycle C représente un cycle benzénique ou un cycle benzénique substitué par un atome d'halogène ou un groupe méthyle.

8. Composé cristallin liquide selon la revendication 6, dans lequel ledit cycle C représente un cycle cyclohexane ou un cycle cyclohexane substitué par un atome d'halogène ou un groupe méthyle.

9. Composition de cristaux liquides comprenant au moins deux constituants dont au moins l'un est un composé cristallin liquide présenté dans l'une quelconque des revendications 1 à 8.
